(19) Europäisches Patentamt
European Patent Office
Office européen des brevets

(11) Veröffentlichungsnummer: **0 022 964**
**B1**

(12) # EUROPÄISCHE PATENTSCHRIFT

(45) Veröffentlichungstag der Patentschrift: **09.02.83**

(21) Anmeldenummer: **80103806.8**

(22) Anmeldetag: **04.07.80**

(51) Int. Cl.³: **C 07 H 19/04**, C 07 H 19/06, C 12 P 19/38, C 07 C 103/52, A 01 N 63/02 // (C12P19/38, C12R1/465)

(54) **Neue Nikkomycine, ein Verfahren zu ihrer Herstellung sowie ihre Verwendung als Schädlingsbekämpfungsmittel.**

(30) Priorität: **12.07.79 DE 2928137**

(43) Veröffentlichungstag der Anmeldung: **28.01.81 Patentblatt 81/4**

(45) Bekanntmachung des Hinweises auf die Patenterteilung: **09.02.83 Patentblatt 83/6**

(84) Benannte Vertragsstaaten: **BE CH DE FR GB IT LI NL SE**

(56) Entgegenhaltungen:
**EP-A-0 013 766**
**DE-A-2 701 890**
**US-A-4 158 608**

(73) Patentinhaber: **BAYER AG, Zentralbereich Patente, Marken und Lizenzen, D-5090 Leverkusen 1, Bayerwerk (DE)**

(72) Erfinder: **Hagenmaier, Hans-Paul, Prof. Dr., Liegnitzer Strasse 8, D-7400 Tübingen (DE)**
Erfinder: **König, Wilfried, Prof. Dr., Grosser Reitweg 36, D-2080 Pinneberg (DE)**
Erfinder: **Zähner, Hans, Prof. Dr., Im Hopfengarten 13, D-7400 Tübingen (DE)**
Erfinder: **Fiedler, Hans-Peter, Dr., Denzenbergstrasse 48, D-7400 Tübingen (DE)**
Erfinder: **Dehler, Wolfgang, Dr., Schönbuchstrasse 47, D-7405 Dettenhausen (DE)**
Erfinder: **Keckeisen, Adelinde, Philosophenweg 79, D-7400 Tübingen (DE)**
Erfinder: **Holst, Hartwig, Dr., Liebigstrasse 18, D-6301 Pohlheim (DE)**
Erfinder: **Zoebelein, Gerhard, Dr., Domblick 46, D-5090 Leverkusen 31 (DE)**

**EP 0 022 964 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue Nikkomycine, ein Verfahren zu ihrer Herstellung sowie ihre Verwendung als Schädlingsbekämpfungsmittel

Die vorliegende Erfindung betrifft neue Nikkomycine, ein mikrobiologisches Verfahren zu ihrer Herstellung aus Streptomyceten-Stämmen sowie ihre Verwendung als Schädlingsbekämpfungsmittel.

Es ist bereits bekanntgeworden, daß Polyoxine als fungitoxische Mittel breite Anwendung im Pflanzenschutz gefunden haben. Ihr Nachteil besteht jedoch in einer Alkali-Labilität und weiterhin darin, daß sie nur als Gemische wechselnder Zusammensetzung vorkommen und somit schlecht exakt dosierbar und standardisierbar sind.

Ferner ist aus der DE-OS 2 537 028 ein als Nikkomycin bezeichnetes Antibioticum bekannt, das eine starke fungizide Wirkung gegen phytopathogene Pilze aufweist.

Es wurde nun gefunden, daß das aus der DE-OS 2 537 028 bekannte Nikkomycin aus mehreren strukturell unterschiedlichen Komponenten besteht und daß die Komponenten oder Komponentengruppen des Nikkomycingemisches als Schädlingsbekämpfungsmittel geeignet sind.

Die Nikkomycine bzw. das aus mehreren Komponenten bestehende Gemisch werden durch submerse Kultur von geeigneten Mikroorganismen in geeigneten Nährlösungen unter geeigneten physikalischen Bedingungen erzeugt. Sie werden aus der Kulturlösung durch Adsorption und Fällung abgetrennt und durch weitere geeignete Methoden angereichert.

Für das Herstellungsverfahren kann der Stamm Streptomyces tendae Ettlinger et al. Tü 901 aus der Ordnung der Actinomycetales, Familie Streptomycetaceae, Gattung Streptomyces eingesetzt werden. Dieser Stamm wurde aus einer Bodenprobe von Nikko/Japan isoliert. Er wurde unter der Nr. CBS 354.75 am 17. Juli 1975 beim Centralbureau voor Schimmelcultures, Baarn/Niederlande, unter der Nr. ATCC 31 160 am 30. Juni 1975 bei der American Type Culture Collection, Rockville, Maryland/USA, und unter der Nr. FRI 3136 beim Fermentation Research Institute, Osaka/Japan, hinterlegt. Dieser Stamm gehört zur Gattung Streptomyces und ist durch folgende Eigenschaften gekennzeichnet:

a) Die Sporen sind ellipsoid. Sie haben die Größe von $0{,}4-0{,}6 \times 1{,}2-1{,}4\,\mu$ und eine glatte Oberfläche.
b) Die Farbe des Luftmycels ist zu Anfang kreideweiß, im ausgereiften Zustand aschgrau (cinereus).
c) Die Sporenketten sind monopodial verzweigt und in lockeren Schrauben und Schlingen angeordnet.
d) Auf Pepton-Eisen-Agar wurde bei 27°C ein schwarzes Pigment gebildet. Der Stamm ist chromogen.

Die zusammengefaßten Bestimmungsmerkmale identifizieren den Stamm Tü 901 als zur Art Streptomyces tendae Ettlinger gehörend.

Für das Verfahren zur Herstellung des Nikkomycin-Gemisches verwendet man Nährmedien, die die üblichen Kohlenstoff- und Stickstoffquellen und die notwendigen Salze enthalten. Als Kohlenstoffquelle können verwendet werden: Kohlenhydrate, insbesondere Polysaccharide, wie z. B. Stärke, Disaccharide, wie z. B. Maltose und Rohrzucker, Monosaccharide, wie z. B. Glucose und Fruktose. Weiterhin können noch verwendet werden Zuckeralkohole, wie z. B. Mannit und Glycerin, außerdem auch natürlich vorkommende Gemische, wie z. B. Malzextrakt. Als Stickstoffquelle können die üblichen Stickstoffquellen Verwendung finden, so z. B. Eiweißstoffe, Eiweißhydrolysate, Aminosäuren, Ammonium-Ionen, Nitrate, natürlich vorkommende komplexe Stoffe, wie Peptone, Casein-Hydrolysate, »Cornsteep-liquor«, Sojamehl, Fleischextrakt und geeignete Mischungen derselben.

Als Hilfsstoffe werden im Nährmedium bevorzugt verwendet die Salze, z. B. Phosphate, Sulfate oder Chloride, des Magnesiums, Eisens, Zinks und Mangans. Die Konzentration dieser Stoffe kann in weiten Grenzen schwanken, zum Teil sind notwendige Konzentrationen in den obengenannten Kohlenstoff- oder Stickstoffquellen oder im verwendeten Wasser als Verunreinigungen enthalten.

Weiterhin können als Hilfsstoffe noch Antischaummittel der verschiedensten Art Verwendung finden, wie z. B. Sojaöl, Polyole oder Silikone. Zur Einhaltung eines gewünschten pH-Bereiches werden Puffer verwendet, auch organische Puffer.

Als wichtigstes Verdünnungsmittel für die Nährmedien ist Wasser zu nennen.

Bei der Durchführung des Herstellungsverfahrens wird im allgemeinen unter aeroben Bedingungen gearbeitet; die Kultur kann gemäß üblichen Methoden, so z. B. unter Verwendung von Schüttelkulturen oder von belüfteten Fermentkulturen, durchgeführt werden. Die Prozentverhältnisse der Nährlösungsbestandteile können in weiten Bereichen schwanken, im allgemeinen machen die Kohlenstoffquellen 1 bis 10%, vorzugsweise 2 bis 5%, aus, die Stickstoffquellen 0,1 bis 4%, vorzugsweise 0,5 bis 2%, aus; die Salze liegen in üblichen Konzentrationen vor, vorzugsweise im Bereich zwischen 0,01 bis 1 Gewichtsprozent. Die Antischaummittel liegen in 0- bis 1%iger Konzentration vor. Die zur Sterilisation angewandten Temperaturen liegen bei 100 bis 140°C, vorzugsweise bei 120 bis 130°C.

Die pH-Werte der wachsenden Kulturen liegen bei 5,5 bis 8, vorzugsweise bei 7 bis 7,5. Die

Züchtungstemperatur kann zwischen 18 und 37°C, vorzugsweise bei 27 bis 30°C liegen. Es hat sich herausgestellt, daß die Menge des sich in der Kulturbrühe anreichernden Antibioticums im allgemeinen ihr Maximum etwa 1 bis 14, vorzugsweise etwa 3 bis 5 Tage nach Züchtungsbeginn erreicht. Der Endpunkt der Bebrütung wird mit Hilfe von biologischen Tests ermittelt, und zwar wird die Wirkung gegen Botrytis cinerea (Testverfahren nach R. Hütter et al., Arch. Mikrobiol. 51, 1—8 [1965]) und Mucor hiemalis (Testverfahren nach Dissertation G. Kirst, Tübingen [1917], ferner nach Kneifel et al., J. Antib. A 27, 20—27 [1974]) ermittelt.

Bei der Durchführung des Herstellungsverfahrens kann zur Aufarbeitung der Kulturlösungen zunächst eine Filtration durchgeführt werden, wobei das Mycel abgetrennt wird. Letzteres kann der Ionenaustausch-Chromatographie an geeigneten Austauschern unterworfen werden. Die Chromatographie kann in Form der Säulenchromatographie oder der präparativen Dünnschichtchromatographie durchgeführt werden. Als Adsorptionsmittel können alle üblichen anorganischen oder organischen Adsorptionsmittel eingesetzt werden, wie z. B. Aluminiumoxid, Kieselgel, Magnesiumsilikat, Aktivkohle, Cellulose, Cellulosederivate, Kunstharze wie Polyamide, Derivate von Polyamiden usw., z. B. acetyliertes Polyamid oder Dextrangele. Als Laufmittel bei der präparativen Dünnschichtchromatographie können die verschiedensten Lösungsmittel oder Lösungsmittelgemische verwendet werden, in welchen das erfindungsgemäße Antibioticum löslich ist. Anschließend kann eine Gel-Chromatographie und eine Reinisolierung an einer weiteren Säule mit anschließender Gefriertrocknung durchgeführt werden.

Das Nikkomycin-Gemisch, das nicht Gegenstand der vorliegenden Erfindung, sondern Gegenstand der DE-OS 2 537 028 ist, kann durch folgende Angaben charakterisiert werden:

a) Löslichkeit und Eigenschaften: Nikkomycin ist ein farbloses, in Wasser und Pyridin sehr gut lösliches, in den sonstigen üblichen organischen Lösungsmitteln unlösliches Substanzgemisch. Es zeigt eine positive Reaktion mit Ninhydrin, Natriummetaperjodat-Benzidin und Kaliumpermanganat. Mit Eisen-(III)-chlorid erhält man eine Gelbfärbung.
b) Das UV-Spektrum und das IR-Spektrum liegen vor (vgl. die entsprechenden Darstellungen in Fig. 1 und 2). In Fig. 1 ist das UV-Spektrum von Nikkomycin, aufgenommen in (a) 1 n-Salzsäure und (b) in 0,1 n-Natronlauge, aufgetragen. Die Ordinate gibt die Extinktion, die Abszisse die Wellenlänge (nm-Einheiten) wieder. In Fig. 2 ist das IR-Spektrum von Nikkomycin, aufgenommen in Kaliumbromid, angegeben. Die Ordinate zeigt die Durchlässigkeit in %, die Abszisse die Wellenzahl ($cm^{-1}$) bzw. Wellenlänge (µm-Einheiten) auf.
c) Mit Hilfe der Massenspektrometrie und des chemischen Abbaus durch saure Hydrolyse konnten Uracil, eine Amino-hexuronsäure und eine neue, einen Pyridinring enthaltende Aminosäure nachgewiesen werden.
d) Papierelektrophorese: Das Antibioticum ist amphoter. Um den pH-Wert von 6 ist die Wanderungsstrecke klein, hier dürfte somit der isoelektrische Punkt liegen. Das Verhalten des Nikkomycin-Gemisches bei der Elektrophorese zeigt die nachfolgende Tabelle, in welcher die Laufstrecken bei der Papierelektrophorese in Abhängigkeit von den pH-Werten des Puffersystems angegeben sind.

| Puffer | pH | Zeit (min) | Laufstrecke (mm) |
|---|---|---|---|
| Pyridin-Eisessig | 3,9 | 60 | −12 |
| Pyridin-Eisessig | 6,1 | 60 | − 1 |
| Barbital*) | 8,9 | 60 | +16 |

*) Barbital ist 5,5-Diäthyl-Barbitursäure

e) Dünnschichtchromatographie: Hierbei treten in allen verwendeten Fließmitteln, die Essigsäure enthalten, drei ninhydrin-positive Flecken auf, wobei jeweils nur ein Fleck mit der im Bioautogramm aktiven Substanz korrespondiert. Diese Tatsache konnte auf die Labilität des Antibioticums in essigsaurer Lösung zurückgeführt werden. In neutralen Fließmitteln zeigt sich jeweils nur ein ninhydrin-positiver Fleck.
f) Als Strukturformel für Nikkomycin wird vorgeschlagen:

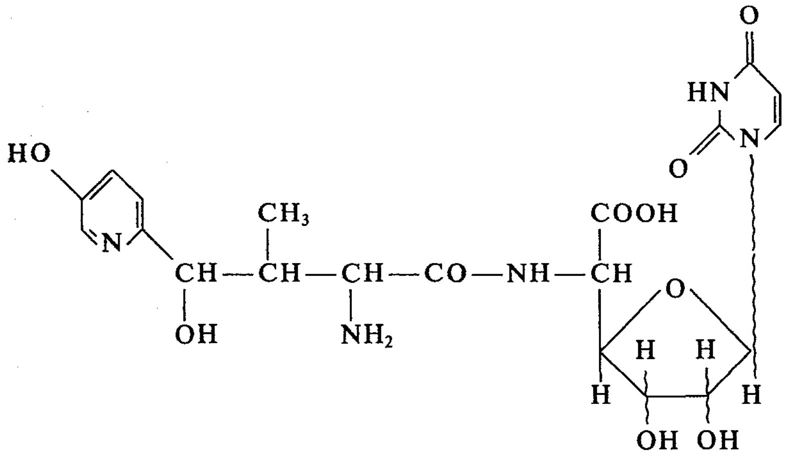

Die Elementaranalyse von Nikkomycin ergab folgende Werte: C 48,21; H 5,08; N 13,58; O 31,58. Daraus errechnet sich eine Elementarzusammensetzung von $C_4H_5NO_2$. Kryoskopisch wurde das Molekulargewicht mit 404 bestimmt. Die hier vorgeschlagene Struktur für Nikkomycin entspricht jedoch dem Molekulargewicht von 495 und der Formel $C_{20}H_{25}N_5O_{10} = (C_4H_5NO_2)_5$. Die daraus errechnete Elementarzusammensetzung ergibt: C 48,49%; H 5,09%; N 14,14%; O 32,29%.

g) Im Gegensatz zu den literaturbekannten Polyoxinen (vgl. J. Am. Chem. Soc. 91, 7490 [1961]), welche dem Nikkomycin am nächsten stehen, fehlt im Nikkomycin der Azacyclobutan-Rest. Die heterocyclische Aminosäure ist in keinem der beschriebenen Polyoxine enthalten.

Die Charakteristik entspricht den Angaben in der DE-OS 2 537 028. Sie gilt, wie inzwischen gefunden wurde, für ein Gemisch strukturell unterschiedlicher Nikkomycine der Formel

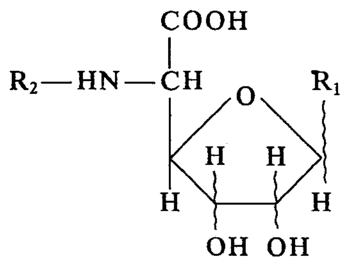

in der $R_1$ die Bedeutung

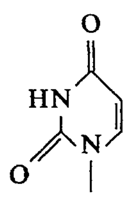

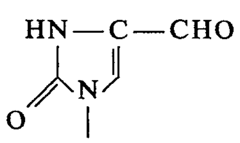

und $R_2$ die Bedeutung Wasserstoff,

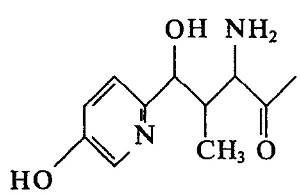

oder

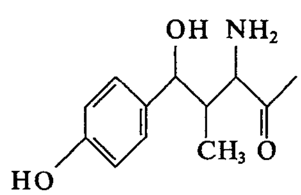

hat.

Insbesondere lassen sich aus dem Nikkomycingemisch die folgenden Wirkstoffkomponenten bzw. Wirkstoffkomponentenpaare

a) 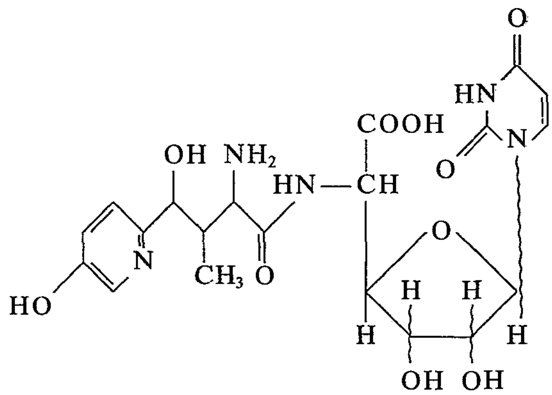 (Z)

und

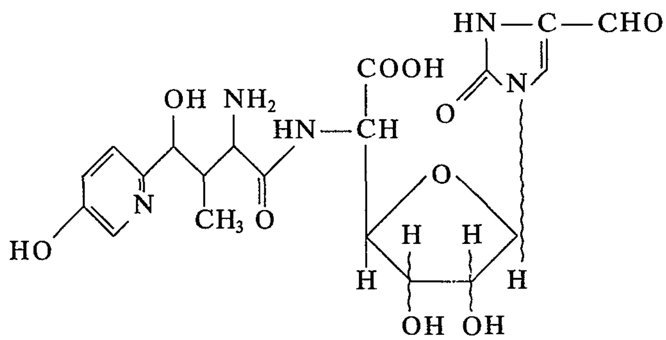 (X)

b) 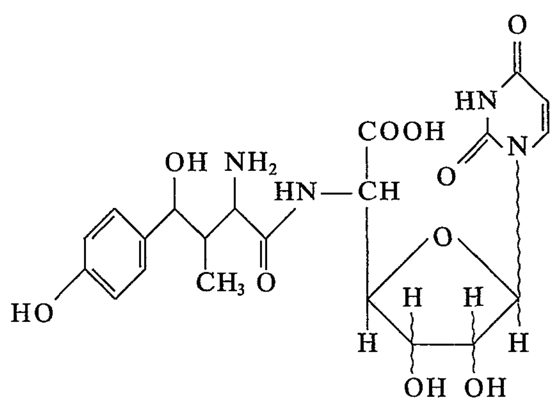 (B)

und

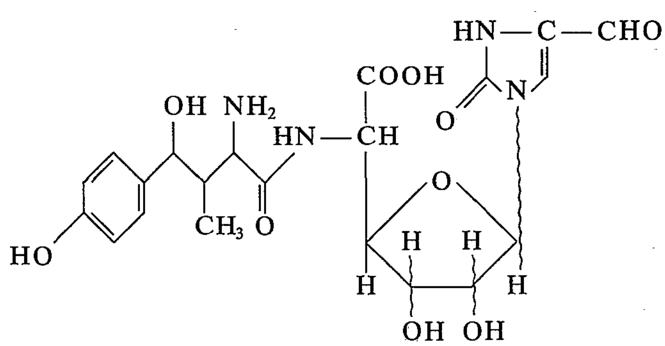 ($B_x$)

c) 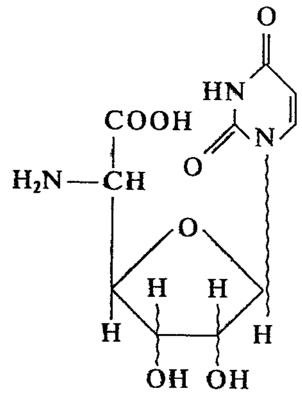 (C)

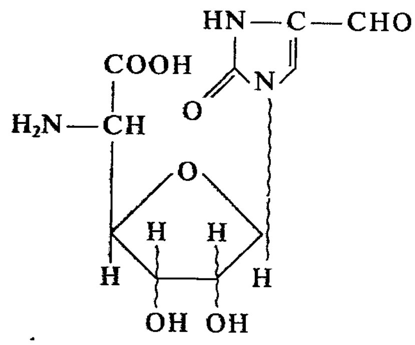 ($C_x$)

isolieren. Diese Wirkstoffkomponenten und Wirkstoffkomponentenpaare eignen sich als Schädlingsbekämpfungsmittel. Sie sind jedoch nicht Gegenstand der vorliegenden Erfindung. Das Nikkomycin-Gemisch und seine Komponenten bzw. Komponentenpaare zeigen überraschenderweise eine ausgezeichnete Wirkung gegen tierische Schädlinge, insbesondere gegen Insekten und Spinnentiere bei guter Pflanzenverträglichkeit und günstiger Warmblütertoxizität.

Diese Wirkstoffe zeigen bei Insekten eine fraßabschreckende, entwicklungsverzögernde und -hemmende Wirkung. Bei Spinnmilben wird durch Anwendung von Nikkomycin-Gemischen bzw. Nikkomycinkomponenten die langsame Ausrottung einer Population bereits nach wenigen Behandlungen erreicht. Insbesondere erfolgt je nach Anwendungszeit eine fast gänzliche Hemmung der Weiterentwicklung in den Ruhestadien Nymphochrysalis, Deutochrysalis und Teleiochrysalis, ein Effekt, der bisher von anderen Naturstoffen oder mikrobiellen Metaboliten nicht bekannt ist. Ein Zusatz anderer Wirkstoffe mit synergistischer oder additiver Wirkung ist nicht erforderlich. Das Nikkomycingemisch bzw. die oben näher beschriebenen Komponenten sind auch gegen phosphorsäureesterresistente Spinnmilben hervorragend wirksam. Die niedrige orale Toxizität für Säugetiere ($>$2000 mg/kg LD 50 bei der Ratte) ist ein weiterer Vorteil.

Es wurden nun aus dem Nikkomycinkomponentengemisch ein Nikkomycingemisch, bestehend aus den Nikkomycinen I und J, sowie ein Nikkomycingemisch, bestehend aus den Nikkomycinen M und N, isoliert, die neu sind und ebenfalls als Schädlingsbekämpfungsmittel geeignet sind.

Die neuen Nikkomycine I, J, M und N weisen folgende Formeln auf, wobei die Schlangenlinien die noch offene Konfiguration andeuten sollen:

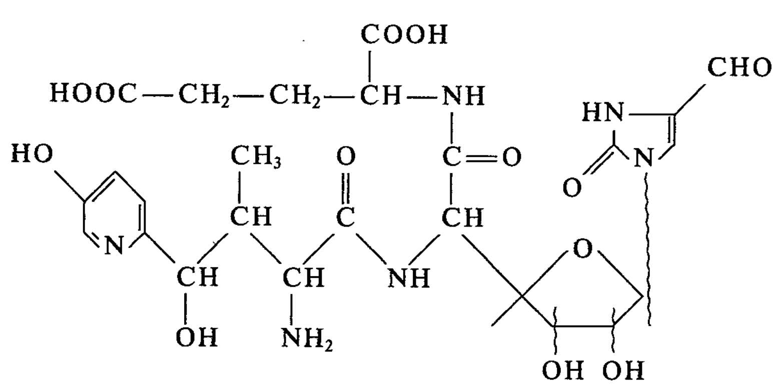

Nikkomycin I

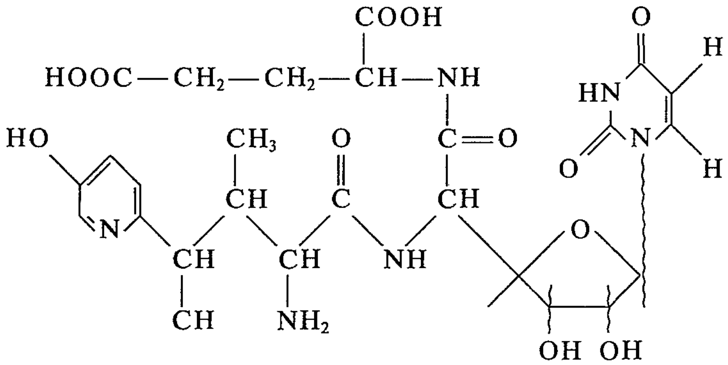

Nikkomycin J

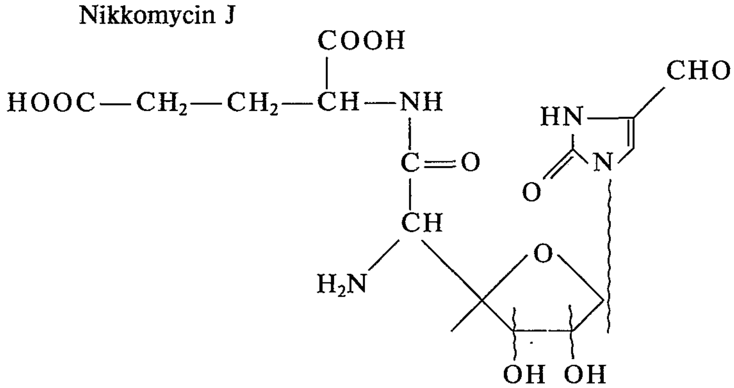

Nikkomycin M

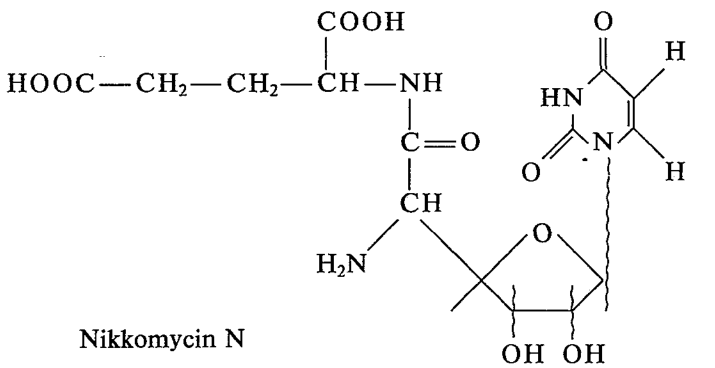

Nikkomycin N

Das Nikkomycin-Gemisch enthält im übrigen noch die Komponenten D und E, deren Struktur im folgenden angegeben wird.

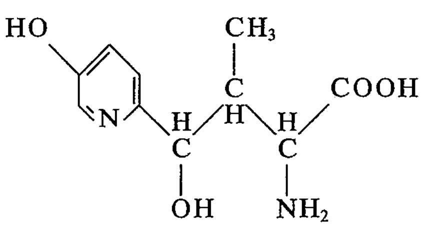

Nikkomycin D

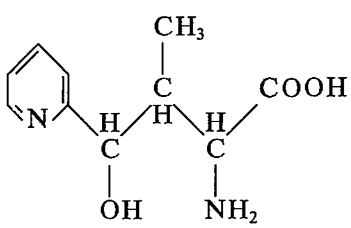

Nikkomycin E

Die Nikkomycine D und E werden nicht beansprucht, da an ihnen bislang keine biologische Aktivität festgestellt worden ist.

Weiterer Gegenstand der Erfindung sind Mittel zur Schädlingsbekämpfung, insbesondere von Insekten und Pilzen. Diese Mittel enthalten die obenerwähnten Mischungen der Nikkomycine I und J sowie der Nikkomycine M und N.

Weiterhin betrifft die Erfindung die Verwendung von Gemischen aus Nikkomycin I und Nikkomycin J bzw. aus Nikkomycin M und Nikkomycin N in und als Schädlingsbekämpfungsmittel.

Die genannten Gemische bestehen jeweils aus etwa 95% I und etwa 5% J bzw. aus etwa 95% M und etwa 5% N, wenn sie nach den weiter unten geschilderten Verfahren hergestellt und isoliert werden.

Die einzelnen Nikkomycine können durch folgende Daten charakterisiert werden.

| UV-Spektren | IR-Spektren | NMR-Spektren |
|---|---|---|
| Fig. 5 $B/B_x$ | Fig. 11 $B/B_x$ | Fig. 17 M/N |
| Fig. 6 $C/C_x$ | Fig. 12 $C/C_x$ | Fig. 18 M |
| Fig. 7 D | Fig. 13 D | |
| Fig. 8 E | Fig. 14 E | |
| Fig. 9 I/J | Fig. 15 I/J | |
| Fig. 10 Z/X | Fig. 16 Z/X | |

$^1$H-NMR-Spektrum der Nikkomycine M, N

Die beiden Singuletts im $^1$H-NMR-Spektrum von Nikkomycin M, N (Abb. 17) bei 9,13 und 7,50 ppm zeigen, daß 4-Formyl-4-imidazolin-2-on als Teilstruktur enthalten ist. Das Dublett bei 5,52 ppm (J = 4,10 Hz) ist dem Proton H-1′ des Nucleosids I zuzuordnen. Die beiden dem Uracil zugehörigen Dubletts bei 7,47 ppm (J = 7,91 Hz) und 5,70 ppm (J = 7,91 Hz) und das dem H-1′ des Nucleosids II zugehörige Dublett bei 5,60 ppm (J = 4,10 Hz) zeigen, daß auch hier — wie bei den Nikkomycinen Z, X und I, J — beide Nucleoside vorliegen. Die Multipletts bei 2,02/1,86 ppm entsprechen den beiden Protonen an C-3″ der Glutaminsäure, die mit den benachbarten Protonen in 4″- und 2″-Stellung koppeln.

Das Triplett bei 2,26 ppm wird durch die beiden H-4″-Protonen verursacht, die mit zwei Protonen in 3″-Stellung koppeln. Die Protonen H-2′, H-3′, H-4′, H-5′ und H-2″ der Glutaminsäure überlagern sich im Bereich von 4,48 bis 4,15 ppm. Das Proton am C-Atom 2″ ist im Vergleich zur freien Glutaminsäure (3,81 ppm) nach tieferem Feld verschoben durch die peptidische Verknüpfung mit dem Nucleosid.

Nachdem Nikkomycin M analog zu Nikkomycin X mit Alkylamine/CPG-1350, einem Glasträger mit 1-Aminopropylgruppierungen, isoliert wurde, fehlten im $^1$H-NMR-Spektrum die Dubletts bei 7,47, 5,70 und 5,70 ppm, die demnach zu Nikkomycin N gehören (Abb. 18).

Dünnschichtchromatographie

Das dünnschichtchromatographische Verhalten der Nikkomycine ist in Tabelle 1 dargestellt.

Bei Fließmittelsystemen, die Eisessig enthalten, tritt während der Chromatographie eine partielle Spaltung der Substanzen in Nucleosid und Aminosäure auf, weshalb nach Besprühen mit Ninhydrin 3 positive Flecken ($Rf_1$, $Rf_2$, $Rf_3$) zu erkennen sind. Nur die Flecken bei $Rf_2$ zeigen das intakte Molekül.

Tabelle 1

Dünnschichtchromatographie der Nikkomycine
a) Laufmittel: Butanol-Pyridin-$H_2O$ (1:1:1) Kieselgel
b) Laufmittel: Propanol-$H_2O$ (8:2) Cellulose
c) Laufmittel: Äthanol-Eisessig-Wasser (4:1:1) Cellulose

| Nikkomycine | Rf-Werte | | | | |
|---|---|---|---|---|---|
| | a | b | c | | |
| | | | $Rf_1$ | $Rf_2$ | $Rf_3$ |
| $B/B_x$ | 0,86 | 0,38 | 0,22 | 0,58 | 0,72 |
| $C/C_x$ | 0,78 | 0,13 | | 0,22 | |
| D | 0,82 | 0,51 | | 0,67 | |
| E | 0,83 | 0,56 | | 0,66 | |
| I/J | 0,85 | 0,25 | 0,22 | 0,30 | 0,62 |
| Z/X | 0,84 | 0,27 | 0,22 | 0,32 | 0,67 |

Papierelektrophorese

Das Verhalten der Nikkomycine bei der Papierelektrophorese in Abhängigkeit von den pH-Werten der Puffersysteme zeigt Tabelle 2.

Tabelle 2

Elektrophoretisches Verhalten in Abhängigkeit vom pH-Wert
a) Pyridin-Acetat-Puffer pH 3,9
b) Pyridin-Acetat-Puffer pH 6,1
c) Borat-HCl-Puffer pH 8,6

| Nikkomycine | Laufstrecke (mm) | | |
|---|---|---|---|
| | a | b | c |
| $B/B_x$ | −3 | 0 | +10 |
| $C/C_x$ | +2 | −2 | +11 |
| D | −4 | −6 | +12 |
| E | −6 | −4 | + 5 |
| I/J | −4 | 0 | +17 |
| Z/X | −6 | 0 | + 2 |

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den obenerwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Pxoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß,

Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und -Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

## Beispiele

### 1) Herstellung des Nikkomycin-Gemisches

Die Nährlösung, in der der Produzentenstamm Streptomyces tendae Tü 901 kultiviert wird, setzt sich aus 2% Sojamehl und 2% Mannit zusammen; der pH wird vor dem Sterilisieren auf pH 7,5 eingesetzt. 10 x 500-ml-Erlenmeyerkolben mit 1 seitlichen Einstich, die je 100 ml Nährlösung enthalten, werden mit dem Produzentenstamm beimpft und 48 Stunden bei 27° C auf einer rotierenden Schüttelmaschine bei 120 Umdrehungen/Minute inkubiert. Mit dieser Vorkultur wird ein 10-l-Fermenter (»New Brunswick«), der 10 l Nährlösung enthält, beimpft, bei 220 Umdrehungen/Minute, 4 l Luftzufuhr/Minute und 27° C 48 Stunden inkubiert. Mit diesem Vorfermenter wird ein 100-l-Fermenter (»New Brunswick«), der 100 l Nährlösung enthält, beimpft, bei 150 Umdrehungen/Minute, 450 l Luftzufuhr/Minute und 27°C 78

Stunden inkubiert.

Die Kultur wird unter Zugabe von 2% Filterhilfsmittel (Hyphlo Supercel®, Johns Mansville) mit einer Filterpresse zuerst über ein Vorklärfilter (C 150, Schenk) und danach über ein Nachklärfilter (U 1000, Schenk) abgepreßt. Das klare Kulturfiltrat wird mit Essigsäure auf pH 4,0 angesäuert und auf eine mit Dowex 50 WX4® (50–100 mesh, $Na^+$-Form) gefüllte Säule (100 x 450 mm) aufgetragen. Die Fließgeschwindigkeit beträgt 10 l/Stunde. Es wird so lange mit entionisiertem Wasser gewaschen, bis die aus der Säule austretende Flüssigkeit völlig farblos ist. Das Antibioticum wird mit je 30 l 0,01 n-Ammoniak und 0,05 n-Ammoniak eluiert. Das biologisch aktive Eluat wird am Rotationsverdampfer von Ammoniak befreit, mit Essigsäure auf pH 4,0 angesäuert und auf eine mit »Amberlite 252«® ($Na^+$-Form) gefüllte Säule (70 x 900 mm) aufgetragen. Die Fließgeschwindigkeit beträgt 5 l/Stunde. Es wird so lange mit entionisiertem Wasser gewaschen, bis die aus der Säule austretende Flüssigkeit völlig farblos ist. Das Antibioticum wird mit 15 l 0,05 n-Ammoniak eluiert. Das biologisch aktive Eluat wird am Rotationsverdampfer vom Ammoniak befreit, auf ein kleines Volumen eingeengt (1 l), mit Essigsäure auf pH 4,0 angesäuert und auf eine mit SP-Sphadex C-25 gefüllte Säule (25 x 850 mm) aufgetragen. Die Fließgeschwindigkeit beträgt 100 ml/Stunde. Mit entionisiertem Wasser wird so lange gewaschen, bis im UV-Durchfluß-Detektor bei 280 nm (Uvicord II, LKB) keine Absorption mehr angezeigt wird. Mit 0,01 m-Pyridin-Acetat-Puffer (pH 4,7) werden Verunreinigungen und mit 0,02 m-Pyridin-Acetat-Puffer (pH 4,7) wird das Antibioticum eluiert. Die biologisch aktiven Fraktionen werden vereinigt, am Rotationsverdampfer vom Puffer befreit und auf ein sehr kleines Volumen eingeengt. Das Konzentrat (10 ml) wird auf eine mit Bio-Gel P 2 (200–400 mesh) gefüllte Säule(25 x 1500 mm) aufgetragen und mit entionisiertem Wasser eluiert. Die Fließgeschwindigkeit beträgt 100 ml/Stunde. Zur Reinheitskontrolle wird mit einem UV-Durchfluß-Detektor bei 280 nm detektiert. Die biologisch aktiven Fraktionen werden vereinigt und in einer Gefriertrocknungsanlage lyophilisiert.

Die Isolierung der Nikkomycin-Komponenten Z und X aus dem so hergestellten Nikkomycin-Gemisch wird im folgenden gezeigt.

2) Isolierung von Nikkomycin Z

100 mg Nikkomycin wurden mit 10 ml einer gesättigten, wäßrigen Dimedonlösung versetzt und 10 Min. auf dem Wasserbad bei 50–70°C erwärmt und anschließend 20 h bei Raumtemperatur stehengelassen. Nach Eindampfen am Rotationsverdampfer wurde der Rückstand in 1 ml $H_2O$ aufgenommen und an einer Säule (0,9 x 60 cm) mit Cellulose (Avicel, Fa. Merck) mit dem Laufmittelsystem A chromatographiert. Das orange-rote Dimedonderivat von X wird zusammen mit überschüssigem Dimedon vor Z eluiert. Die Fraktionen mit Z wurden vereinigt und an derselben Säule rechromatographiert.

Ausb. 11,4 mg Z. Laufmittel A: Äthanol/Essigsäure/Wasser (4 : 1 : 1) Volumenteile.

3) Isolierung von Nikkomycin X

19 g Nikkomycin wurden in 75 ml $H_2O$ gelöst und 9 g Alkylamine/CPG-1350 (Pierce Chem. Comp.) zugegeben. Nach dem Entgasen im Vakuum ließ man 24 h bei Raumtemperatur stehen. Das Trägermaterial wurde abfiltriert, mit 800 ml $H_2O$ ausgewaschen und mit 100 ml 5% Essigsäure 30 Min. bei Raumtemperatur behandelt. Das Filtrat wurde lyophilisiert. Man erhielt einen Rückstand von 116 mg, der anschließend an LiChroprep RP-8 mit Methanol/$H_2O$ (30 : 70) chromatographiert wurde. Ausbeute an X (nach Lyophilisieren) 18 mg.

4) Bestimmung des Verhältnisses von Nikkomycin X und Z

a) Mittels $^1$H-NMR-Spektroskopie

Nikkomycin Z läßt sich im $^1$H-NMR-Spektrum durch die beiden miteinander koppelnden Protonen in Stellung 5 ($\delta = 5,91$; d; 8 Hz) und Stellung 6 ($\delta = 7,59$; d; 8 Hz) erkennen. Im Nikkomycin X fehlen diese beiden Resonanzen. Dafür treten zwei Singuletts bei $\delta = 7,69$ und $\delta = 9,33$ auf. Durch Vergleich der Integrale dieser Signale ist eine Bestimmung des Verhältnisses von Nikkomycin X und Z in der Mischung möglich.

b) Mittels Hochdruckflüssigkeitschromatographie

An einer RP-18-Säule (Merck, 10 u. 250 x 4,6 mm) mit einer 0,005molaren Lösung von 1-Hexansulfonsäure in Methanol/$H_2O$/Essigsäure (14/84/2) als Elutionsmittel lassen sich die beiden

Nikkomycine X und Z auftrennen und mittels UV-Detektion bei 280 nm quantitativ bestimmen. Diese Bestimmung kann direkt aus dem Kulturfiltrat erfolgen.

## Weitere Verfahren zur Isolierung und Charakterisierung der Nikkomycine

Im folgenden Schema sind die Verfahren zur Herstellung von Reinsubstanzen aus Kulturen von Streptomyces tendae dargestellt.

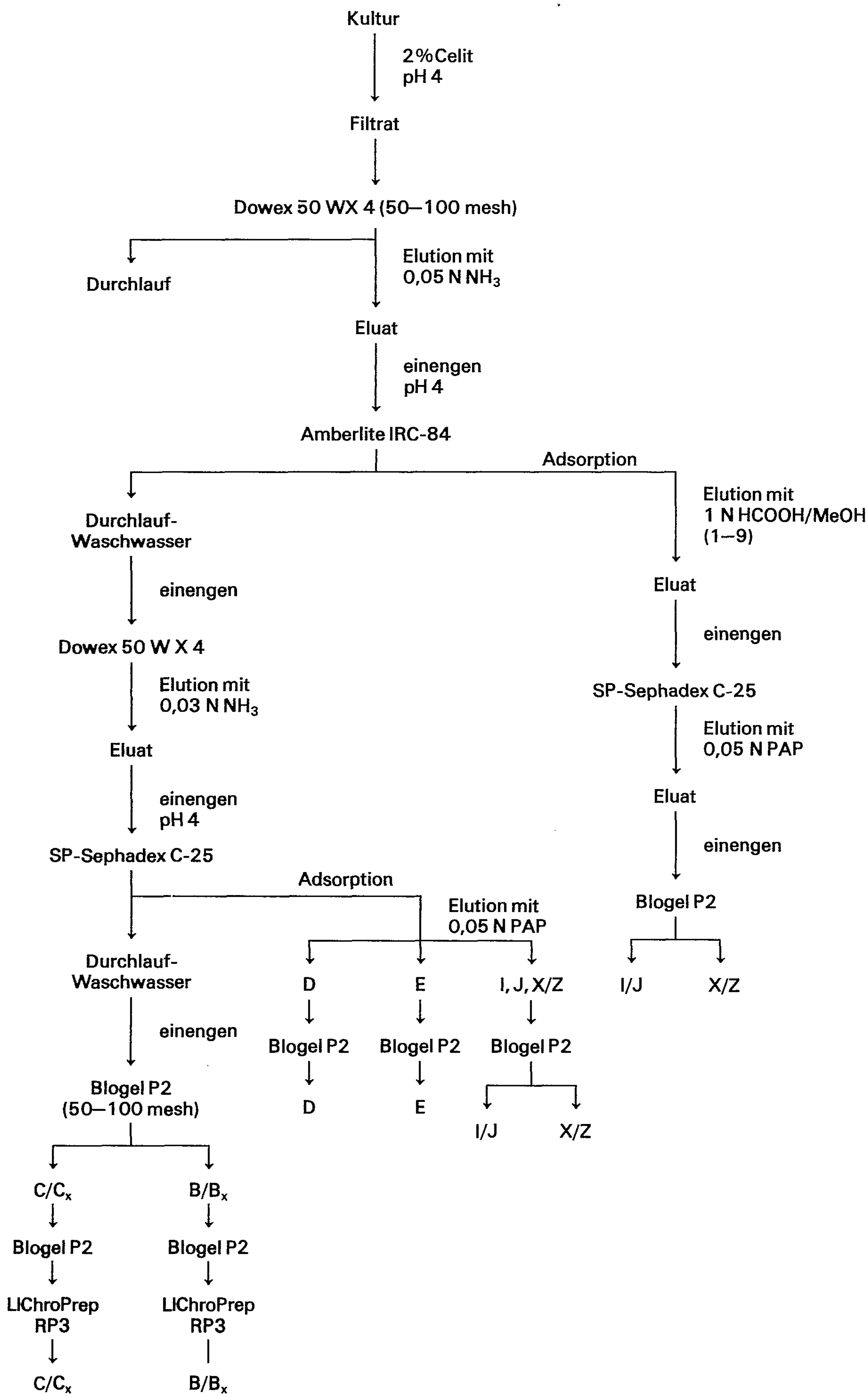
Kultur
2% Celit
pH 4
Filtrat
Dowex 50 WX 4 (50–100 mesh)
Durchlauf
Elution mit
0,05 N $NH_3$
Eluat
einengen
pH 4
Amberlite IRC-84
Adsorption
Durchlauf-
Waschwasser
einengen
Dowex 50 W X 4
Elution mit
0,03 N $NH_3$
Eluat
einengen
pH 4
SP-Sephadex C-25
Adsorption
Elution mit
0,05 N PAP
Durchlauf-
Waschwasser
einengen
Blogel P2
(50–100 mesh)
C/$C_x$
B/$B_x$
Blogel P2
Blogel P2
LIChroPrep
RP3
LIChroPrep
RP3
C/$C_x$
B/$B_x$
D
E
I, J, X/Z
Blogel P2
Blogel P2
Blogel P2
D
E
I/J
X/Z
Elution mit
1 N HCOOH/MeOH
(1–9)
Eluat
einengen
SP-Sephadex C-25
Elution mit
0,05 N PAP
Eluat
einengen
Blogel P2
I/J
X/Z

5) Isolierung der Nikkomycine I/J, Z und X

Die Kultur (20-l-Fermenter) wurde nach Zusatz von 2% Celit mit einer Mehrschichtenfilterpresse über die Filterschicht C 150 (Schenk Filterbau, Schwäbisch Gmünd) vorgeklärt. Das Kulturfiltrat wurde mit Essigsäure auf pH 4 eingestellt und mit einer Mehrschichtenfilterpresse über die Filterschicht U 1000 nachgeklärt.

Das klare Kulturfiltrat wird auf eine mit Dowex 50WX4 (50–100 mesh, $Na^+$-Form) gefüllte Säule aufgetragen (Bettvolumen 3 l, Fließgeschwindigkeit 7 l/h). Mit entionisiertem Wasser wäscht man so lange, bis die aus der Säule austretende Flüssigkeit völlig farblos ist. Verunreinigungen werden mit 0,01 N $NH_3$-Lösung und das Nikkomycingemisch mit 0,05 N $NH_3$-Lösung eluiert. Das biologisch aktive Eluat wird im Vakuum vom Ammoniak befreit und mit Ameisensäure auf pH 5,5 angesäuert.

Das so behandelte Eluat wird auf eine mit Amberlite IRC-84 ($Na^+$-Form) gefüllte Säule aufgetragen (Bettvolumen 2 l, Fließgeschwindigkeit 5 l/h). Die Säule wird mit der dreifachen Menge des Auftragsvolumens mit Wasser gewaschen. Das an IRC-84 gebundene Material eluiert man mit einem 1N HCOOH/MeOH-Gemisch (1+9) und engt es im Vakuum auf ein kleines Volumen ein.

Das Konzentrat wird auf eine mit SP-Sephadex C-25 (Pyridinium-Form) gefüllte Säule aufgetragen (Bettvolumen 1 l, Fließgeschwindigkeit 100 ml/h). Mit entionisiertem Wasser wird so lange gewaschen, bis im UV-Detektor (Unicord II, LKB, Bromma, Schweden) bei 280 nm keine Absorption mehr angezeigt wird. Mit 0,01 N Pyridin-Acetat-Puffer werden Verunreinigungen und mit 0,05 N Pyridin-Acetat-Puffer (pH 4,7) werden die Nikkomycine I/J, X und Z eluiert.

Die biologisch aktiven Fraktionen werden vereinigt, im Vakuum vom Puffer befreit, auf ein kleines Volumen eingeengt und in einer Gefriertrocknungsanlage lyophilisiert. Das so gewonnene Produkt wird in kleinen Portionen (300–400 mg) gelchromatographisch an Biogel P2 (200–400 mesh, Bettvolumen 1 l, Fließgeschwindigkeit 60 ml/h) gereinigt. Man erhält eine Auftrennung in Nikkomycin I/J und das natürlich anfallende Nikkomycin-Z/X-Gemisch. Die entsprechenden Fraktionen werden vereinigt, eingeengt und lyophilisiert.

Das anfallende Nikkomycin-Z/X-Gemisch bestand, bestimmt nach der Dimedon-Reaktion, aus 90% Nikkomycin X und 10% Nikkomycin Z. Die Ausbeute aus einem 20-l-Fermenter betrug 25% der im Kulturfiltrat vorhandenen Menge, so daß bei einer Produktion von 1,3 g/l ca. 5 g reines Nikkomycin-Z/X-Gemisch ioliert werden konnte.

Reines Nikkomycin I/J ließ sich mit einer Ausbeute von etwa 0,8 g isolieren.

Fig. 3: Elutionsdiagramm der Biogel-P2-Säule
______ UV-Absorption bei 280 nm
.......... Aktivität gegen Mucor hiemalis (±)
Peak 1: gelber Farbstoff; Peak 2: Nikkomycin I/J;
Peak 3: Nucleosidteil von Nikkomycin Z/X;
Peak 4: Aminosäureteil von Nikkomycin Z/X;
Peak 5: Nikkomycin Z/X

6) Isolierung der Nikkomycine $B/B_x$ und $C/C_x$

Der Durchlauf und das Waschwasser der Amberlite IRC-84-Säule (ca. 10% der auf die Säule aufgebrachten Aktivität) werden vereinigt und auf eine mit Dowex 50WX4 ($Na^+$-Form, 50–100 mesh) gefüllte Säule aufgetragen (Bettvolumen 700 ml, Fließgeschwindigkeit 3 l/h). Mit 0,01 N $NH_3$-Lösung werden Verunreinigungen, mit 0,03 N $NH_3$-Lösung das Nikkomycingemisch eluiert. Die aktiven Eluate werden vereinigt, am Rotationsverdampfer vom Ammoniak befreit, der pH mit Essigsäure auf 4,7 eingestellt und auf eine mit SP-Sephadex C-25 gefüllte Säule (Bettvolumen 500 ml, Fließgeschwindigkeit 100 ml/h) aufgetragen. Die Säule wird mit entionisiertem Wasser so lange gewaschen, bis im UV-Detektor bei 280 nm keine Absorption mehr angezeigt wird. Im Durchlauf und im Waschwasser sind die Nikkomycine $B/B_x$ und $C/C_x$ enthalten. Der Durchlauf und das Waschwasser werden vereinigt, auf ein sehr kleines Volumen eingeengt und über Biogel P2 (50–100 mesh, Bettvolumen 1 l, Fließgeschwindigkeit 150 ml/h) chromatographiert. Man erhält eine Auftrennung in Nikkomycin $C/C_x$ und $B/B_x$. Überlappende Fraktionen werden rechromatographiert. Die weitere Reinigung der Nikkomycine erfolgt dann durch Gelchromatographie an Biogel P2 (100–200 mesh, Bettvolumen 500 ml, Fließgeschwindigkeit 80 ml/h). Zur Gewinnung von Reinsubstanz wird eine Reversed-phasechromatographie an LiChroPrep RP8 (Korngröße 20–40 μ, Bettvolumen 250 ml, Fließgeschwindigkeit 60 ml/h) durchgeführt. Als Laufmittel dient entionisiertes Wasser.

Die Ausbeute (20-l-Fermenter) an Nikkomycin-$C/C_x$-Gemisch betrug 350 mg. Das Gemisch setzt sich aus 10% Nikkomycin C und 90% Nikkomycin $C_x$ zusammen.

An Nikkomycin $B/B_x$ ließen sich 120 mg isolieren. Im Nikkomycin-$B/B_x$-Gemisch ist die Komponente Nikkomycin B nur in Spuren vorhanden.

7) Isolierung von Nikkomycin D, E, I/J und Z/X

An der bei 6) beschriebenen SP-Sephadex C-25-Säule werden die Nikkomycine D, E, I/J und Z/X gebunden. Mit 0,03 N-Pyridin-Acetat-Puffer werden die Nikkomycine D und E in getrennten Peaks und die Nikkomycine I/J, Z und X in einem gemeinsamen Peak eluiert. Die Nikkomycine D und E können nach Entfernen des Pyridin-Acetat-Puffers und Einengen im Vakuum an Biogel P2 (100 – 200 mesh, Bettvolumen 500 ml, Fließgeschwindigkeit 80 ml/h) gereinigt werden.

Die Ausbeute an Nikkomycin D betrug 180 mg, an Nikkomycin E 130 mg.

Die Nikkomycine I/J und Z/X können wie unter 5) beschrieben getrennt und gereinigt werden.

Mengenmäßig ergeben die auf diesem Weg isolierten Nikkomycine I/J und Z/X etwa ein Zehntel der unter 5) isolierten Ausbeute.

Fig. 4: Elutionsdiagramm der SP-Sephadex C-25-Säule mit 0,03 N PAP
______ UV-Absorption bei 280 nm
.............. Aktivität gegen Mucor hiemalis (±)
Peak 1: Nikkomycin D
Peak 2: Nikkomycin E
Peak 3: Nikkomycine I/J und Z/X

8) Isolierung von Nikkomycin M/N

Die Nikkomycine M/N entstehen aus dem Nikkomycin-I/J-Gemisch durch Hydrolyse.

14 g Nikkomycinmischung (ca. 30%ig nach biol. Aktivität) werden mit 200 ml Wasser und 200 ml 0,02 M Phosphatpuffer pH 7,3 versetzt und 8 Tage bei 37°C stehengelassen. Nach dem Ansäuern mit Essigsäure auf pH 2,2 wird die Lösung auf eine Säule, gefüllt mit AG 50 W-X8 ($H^+$-Form; 50 – 100 mesh; Biorad; 90 × 4 cm), aufgetragen. Es wird mit einem linearen Gradienten aus je 2 l Pyridinacetatpuffer (0,2 M; pH 3,1 und 2 M; pH 5,0) eluiert. Zuerst werden die Nucleoside C/$C_x$, Nikkomycin M/N und Nikkomycin E zusammen eluiert, dann Nikkomycin D.

Die Fraktionen mit Nikkomycin D wurden eingeengt und in Wasser aufgenommen. Beim Neutralisieren entstand ein feinpulvriger weißer Niederschlag, der abzentrifugiert und aus heißem Wasser umkristallisiert wurde.

Die Fraktionen aus Nucleosiden, Nikkomycin M/N und Nikkomycin E wurden eingeengt, in Wasser aufgenommen und in 2 Portionen auf eine Cellulosesäule (2,5 × 100 cm), gefüllt mit Avicel, Fa. Merck, aufgetragen und mit Äthanol/Eisessig/Wasser (4 : 1 : 1) in dieser Reihenfolge eluiert: Nikkomycin E, Nikkomycin M/N, Nucleosid C, Nucleosid $C_x$.

Ausbeuten:

| | |
|---|---|
| Nikkomycin D: | 1,05 g (krist.), 817 mg (gefriergetrocknet) |
| Nikkomycin E: | 405 mg |
| Nikkomycin M/N: | 1,27 g (gefriergetrocknet) |
| Nucleosid C: | 284 mg (krist.) + 317 mg (gefriergetrocknet) |
| Nucleosid C/$C_x$: | 800 mg (gefriergetrocknet) |

In den obigen Herstellungsbeispielen werden, zum Teil mit Warenbezeichnungen, Reagenzien, Hilfsmittel und technische Geräte folgender Firmen genannt:

a) Fermenter der Firma New Brunswick Scientific Corporation Inc., New Brunswick, New Jersey/USA
b) Hyphlo Supercel®, Firma Johns, Mansville, Cal., USA
c) Filterpressen C 150 und U 1000 der Firma Schenck, Filterbau, Schwäbisch-Gmünd, BRD
d) Dowex®, Warenzeichen der Firma Dow Chemical Co., Midland, Michigan/USA
e) Amberlite®, Warenzeichen der Firma Rohm und Haas Co., Philadelphia, Pennsylvania/USA
f) SP-Sephadex®, Warenzeichen der Firma Pharmacia Fine Chemicals, Upsala/Schweden
g) Uvicord® II, Firma LKB, Bromma, Schweden

Alle Prozentangaben bedeuten Gewichtsprozente.

**Patentansprüche**

1. Nikkomycingemisch, bestehend aus Nikkomycin I der Formel

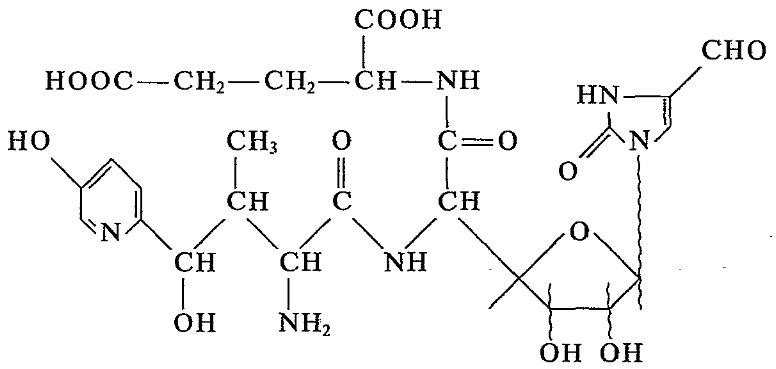

(I)

und aus Nikkomycin J der Formel

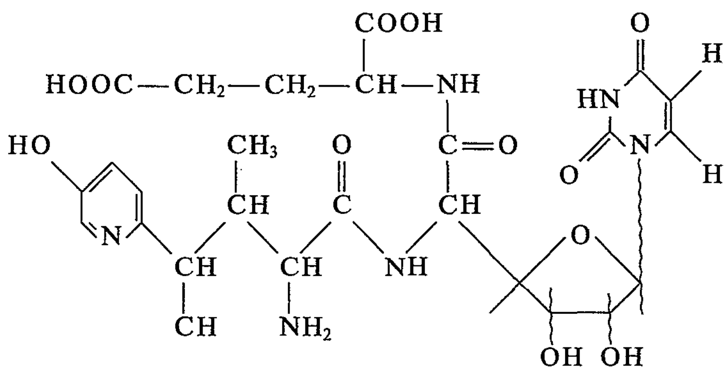

(J)

sowie Nikkomycingemisch, bestehend aus Nikkomycin M der Formel

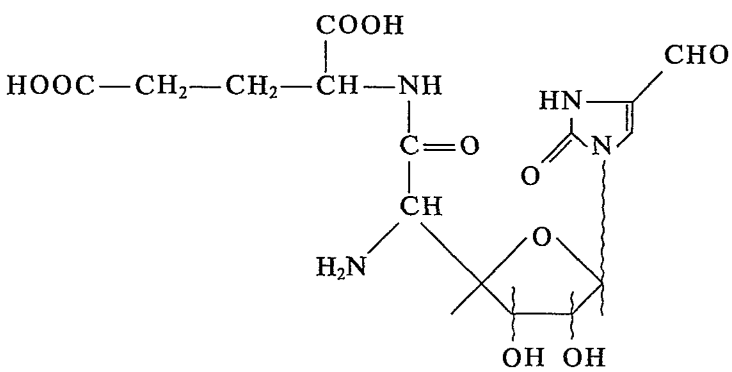

(M)

und aus Nikkomycin N der Formel

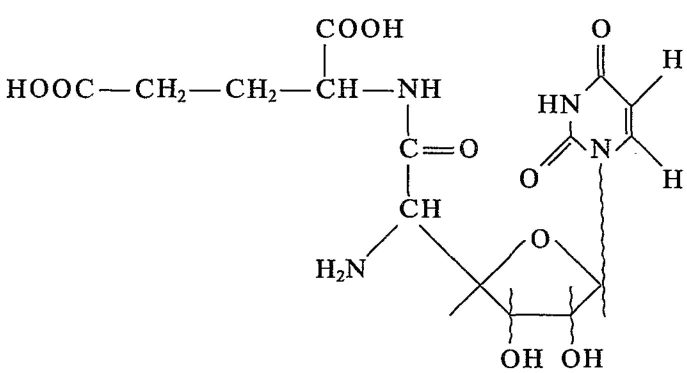

(N)

wobei die Schlangenlinien in den obigen Formeln die noch offene Konfiguration andeuten sollen.

2. Schädlingsbekämpfungsmittel, enthaltend das Nikkomycingemisch aus Nikkomycin I und Nikkomycin J und/oder das Nikkomycingemisch aus Nikkomycin M und Nikkomycin N gemäß Anspruch 1.

3. Verwendung des Nikkomycingemisches aus Nikkomycin I und Nikkomycin J und/oder des Nikkomycingemisches aus Nikkomycin M und Nikkomycin N nach Anspruch 1 zur Schädlingsbekämpfung, insbesondere zur Bekämpfung von Insekten, Spinnentieren und Pilzen.

4. Verfahren zur Bekämpfung von Schädlingen, dadurch gekennzeichnet, daß man das Nikkomycingemisch aus Nikkomycin I und Nikkomycin J und/oder das Nikkomycingemisch aus Nikkomycin M und Nikkomycin N gemäß Anspruch 1 auf die genannten Schädlinge bzw. deren Lebensraum einwirken läßt.

5. Verfahren zur Herstellung von Schädlingsbekämpfungsmitteln, dadurch gekennzeichnet, daß man das Nikkomycingemisch aus Nikkomycin I und Nikkomycin J und/oder das Nikkomycingemisch aus Nikkomycin M und Nikkomycin N gemäß Anspruch 1 mit Streckmitteln und/oder oberflächenaktiven Mitteln mischt.

6. Verfahren zur Herstellung der Nikkomycingemische aus Nikkomycin I und Nikkomycin J sowie aus Nikkomycin M und Nikkomycin N nach Anspruch 1 durch aerobes Züchten von Mikroorganismen, dadurch gekennzeichnet, daß man Stämme der Ordnung Actinomycetales, der Familie Streptomycetaceae, insbesondere der Gattung Streptomyces, in wäßrigem Nährmedium nach üblichen Methoden züchtet und die Nikkomycingemische anschließend isoliert.

7. Verfahren zur Herstellung der Nikkomycingemische aus Nikkomycin I und Nikkomycin J sowie aus Nikkomycin M und Nikkomycin N nach Anspruch 1 durch aerobes Züchten von Mikroorganismen, dadurch gekennzeichnet, daß man den Stamm Streptomyces tendae, Ettlinger et al. Tü 901, hinterlegt unter der Nummer CBS 354.75 beim Centralbureau voor Schimmelkultures, Baarn/Niederlande, unter der Nummer ATCC 31 160 bei der American Type Culture Collection, Rockville, Maryland/USA, und unter der Nummer FRI 3136 beim Fermentation Research Institut Osaka/Japan, in wäßrigem Medium nach üblichen Methoden züchtet und die Nikkomycingemische anschließend isoliert.

## Claims

1. A nikkomicin mixture consisting of nikkomicin I of the formula

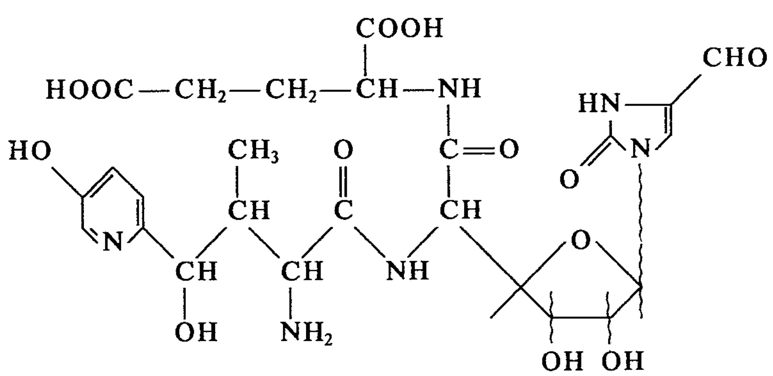

(I)

and of nikkomicin J of the formula

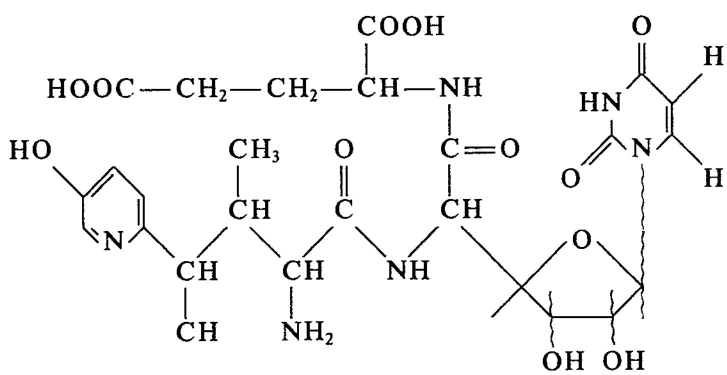

(J)

and a nikkomicin mixture consisting of nikkomicin M of the formula

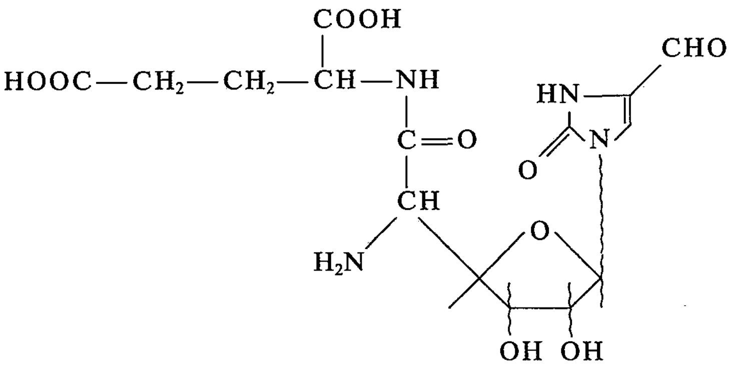

(M)

and of nikkomicin N of the formula

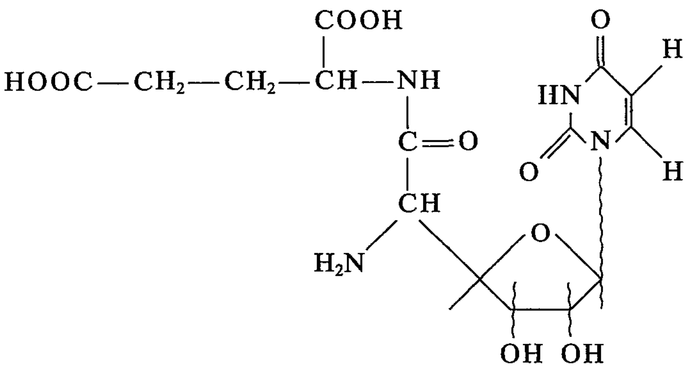

(N)

the wavy lines in the above formulae being intended to indicate the configuration which is still open.

2. Pest-combating agent containing the nikkomicin mixture consisting of nikkomicin I and nikkomicin J, and/or the nikkomicin mixture consisting of nikkomicin M and nikkomicin N according to Claim 1.

3. Use of the nikkomicin mixture consisting of nikkomicin I and nikkomicin J and/or the nikkomicin mixture consisting of nikkomicin M and nikkomicin N according to Claim 1, for combating pests, in particular for combating insects, arachnida and fungi.

4. Process for combating pests, characterised in that the nikkomicin mixture consisting of nikkomicin I and nikkomicin J, and/or the nikkomicin mixture consisting of nikkomicin M and nikkomicin N according to Claim 1 are allowed to act on the stated pests or on their habitat.

5. Process for the preparation of pest-combating agents, characterised in that the nikkomicin mixture consisting of nikkomicin I and nikkomicin J, and/or the nikkomicin mixture consisting of nikkomicin M and nikkomicin N according to Claim 1 are mixed with extenders and/or surface-active agents.

6. Process for the preparation of the nikkomicin mixtures consisting of nikkomicin I and nikkomicin J and of nikkomicin M and nikkomicin N according to Claim 1 by aerobic culture of micro-organisms, characterised in that strains of the order Actinomycetales, of the family Streptomycetaceae, in particular of the genus Streptomyces, are cultured in an aqueous nutrient medium by customary methods and the nikkomicin mixtures are then isolated.

7. Process for the preparation of the nikkomicin mixtures consisting of nikkomicin I and nikkomicin J and of nikkomicin M and nikkomicin N according to Claim 1 by aerobic culture of micro-organisms, characterised in that the strain Streptomyces tendae, Ettlinger et al. Tü 901, deposited under the number CBS 354.75 in the Centralbureau voor Schimmelkultures (Central Bureau of Mould Cultures), Baarn/Netherlands, under the number ATCC 31 160 in the American Type Culture Collection, Rockville, Maryland/USA, and under the number FRI 3136 in the Fermentation Research Institute Osaka/Japan, is cultured in an aqueous medium by customary methods, and the nikkomicin mixtures are then isolated.

**Revendications**

1. Mélange de nikkomycines, consistant en nikkomycine I de formule

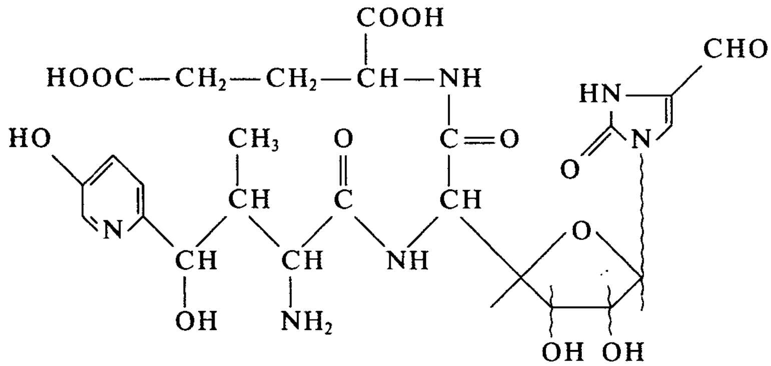

(I)

et en nikkomycine J de formule

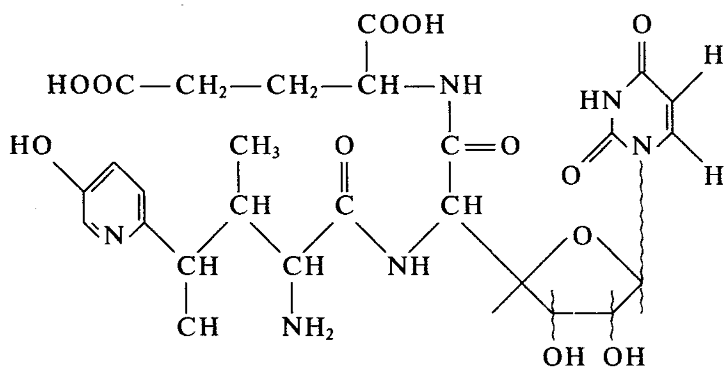

(J)

ainsi que mélanges de nikkomycines consistant en nikkomycine M de formule

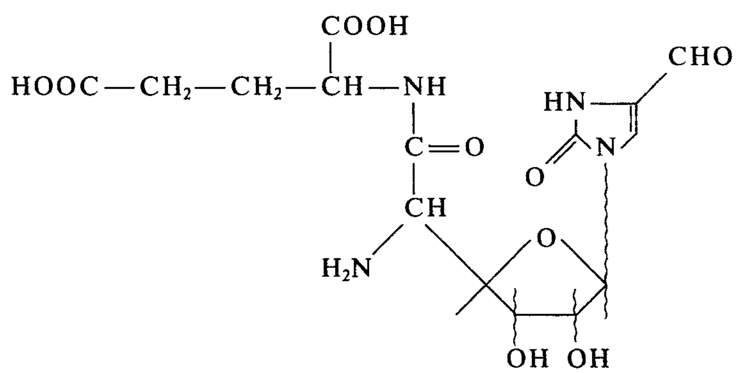

(M)

et en nikkomycine N de formule

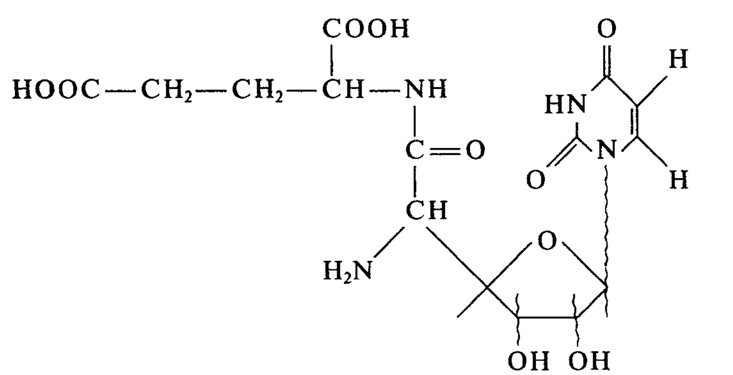

(N)

dans lesquelles les traits ondulés désignent la configuration encore ouverte.

2. Agent de lutte contre les parasites, contenant le mélange de nikkomycines consistant en nikkomycine I et nikkomycine J et/ou le mélange de nikkomycine consistant en nikkomycine M et nikkomycine N selon la revendication 1.

3. Utilisation du mélange de nikkomycines consistant le nikkomycine I et le nikkomycine J et/ou du mélange de nikkomycines consistant en nikkomycine M et nikkomycine N selon la revendication 1 pour la lutte contre les parasites, en particulier contre les insectes, les araignées et les champignons.

4. Procédé de lutte contre les parasites, caractérisé en ce que l'on fait agir sur les parasites mentionnés ou leur espace vital le mélange de nikkomycines consistant en nikkomycine I et nikkomycine J et/ou le mélange de nikkomycines consistant en nikkomycine M et nikkomycine N selon la revendication 1.

5. Procédé pour la préparation d'agents de lutte contre les parasites, caractérisé en ce que l'on mélange le mélange de nikkomycines consistant en nikkomycine I et nikkomycine J et/ou le mélange de nikkomycines consistant en nikkomycine M et nikkomycine N selon la revendication 1 avec des agents diluant et/ou des agents tensioactifs.

6. Procédé pour la préparation des mélanges de nikkomycines consistant en nikkomycine I et nikkomycine J ainsi qu'en nikkomycine M et nikkomycine N selon la revendication 1 par culture aérobie de micro-organismes, caractérisé en ce que l'on cultive des souches de l'ordre des actinomycetales, de la famille des Streptomycetaceae, en particulier de l'espèce Streptomyces, par des techniques habituelles en milieu nutritif aqueux et on isole ensuite les mélanges de nikkomycines.

7. Procédé pour la préparation des mélanges de nikkomycines consistant en nikkomycine I et nikkomycine J ainsi qu'en nikkomycine M et nikkomycine N selon la revendication 1 par culture aérobie de micro-organisme, caractérisé en ce que l'on cultive en milieu aqueux selon les techniques habituelles la souche Streptomyces tendae, Ettlinger et al. Tü 901, déposée sous le n° CBS 354.75 au Centralbureau voor Schimmelkultures, Baarn, Pays-Bas, sous le n° ATCC 31 160 à l'American Type Culture Collection, Rockville, Maryland, EUA, et sous le n° FRI 3136 au Fermentation Research Institut, Osaka, Japon, et on isole les mélanges de nikkomycines.

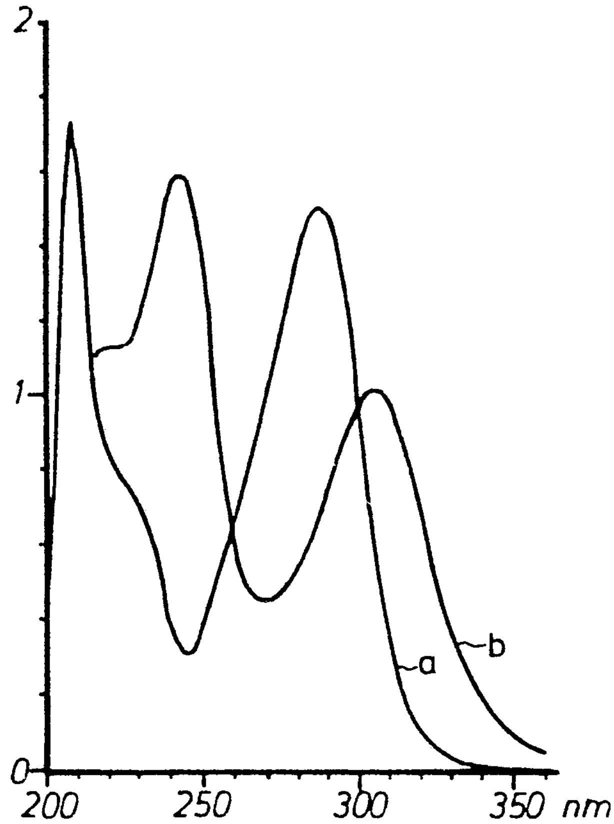

2
1
0
200
250
300
350 nm
a
b


FIG. 1

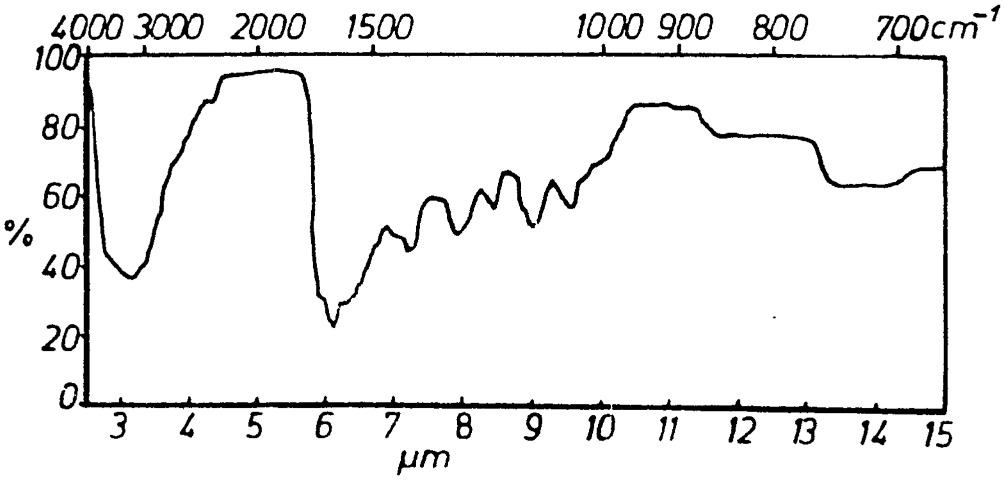

4000 3000 2000 1500 1000 900 800 700cm⁻¹
100
80
60
%
40
20
0
3 4 5 6 7 8 9 10 11 12 13 14 15
μm


FIG. 2

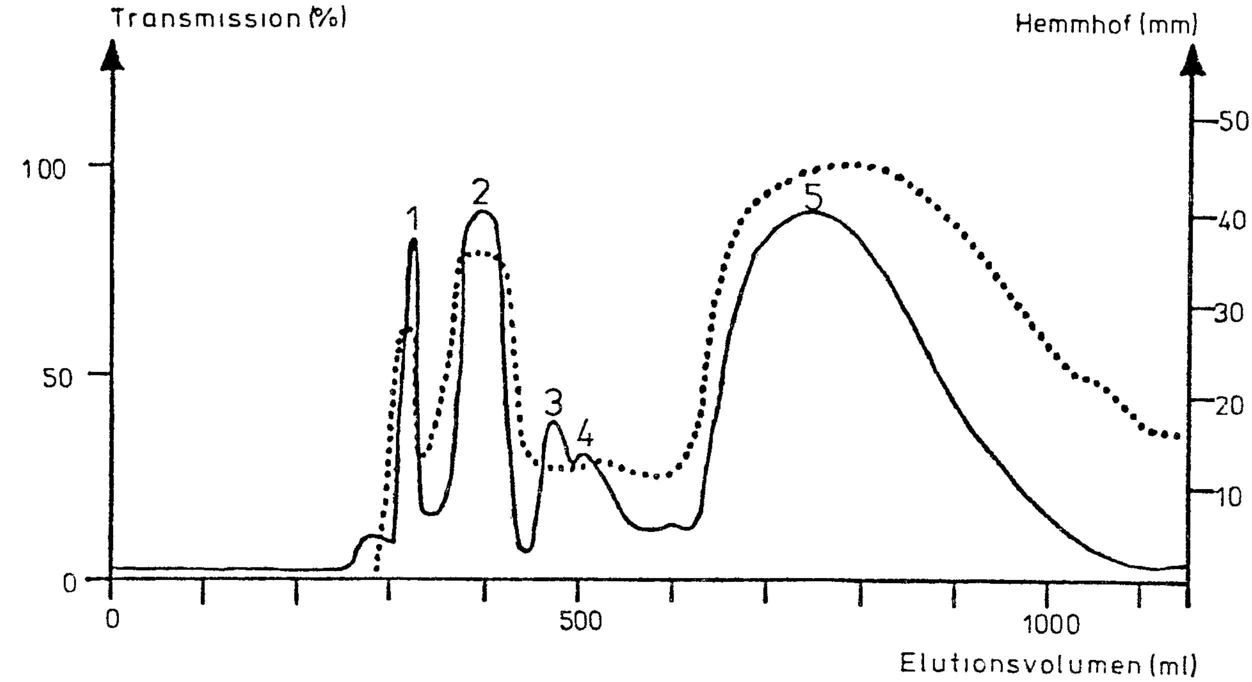
Transmission (%)
Hemmhof (mm)
100
50
0
50
40
30
20
10
1
2
3
4
5
0
500
1000
Elutionsvolumen (ml)

FIG. 3

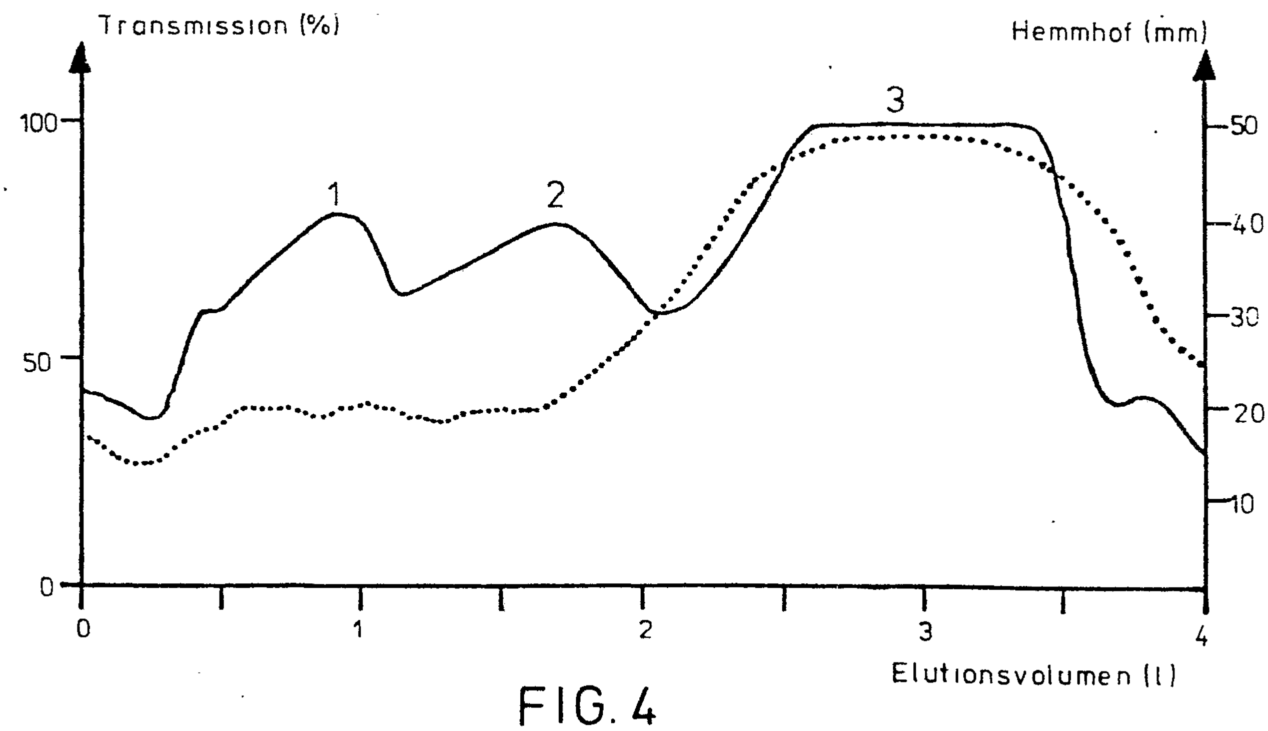
Transmission (%)
Hemmhof (mm)
100
50
0
50
40
30
20
10
1
2
3
0
1
2
3
4
Elutionsvolumen (l)

FIG. 4

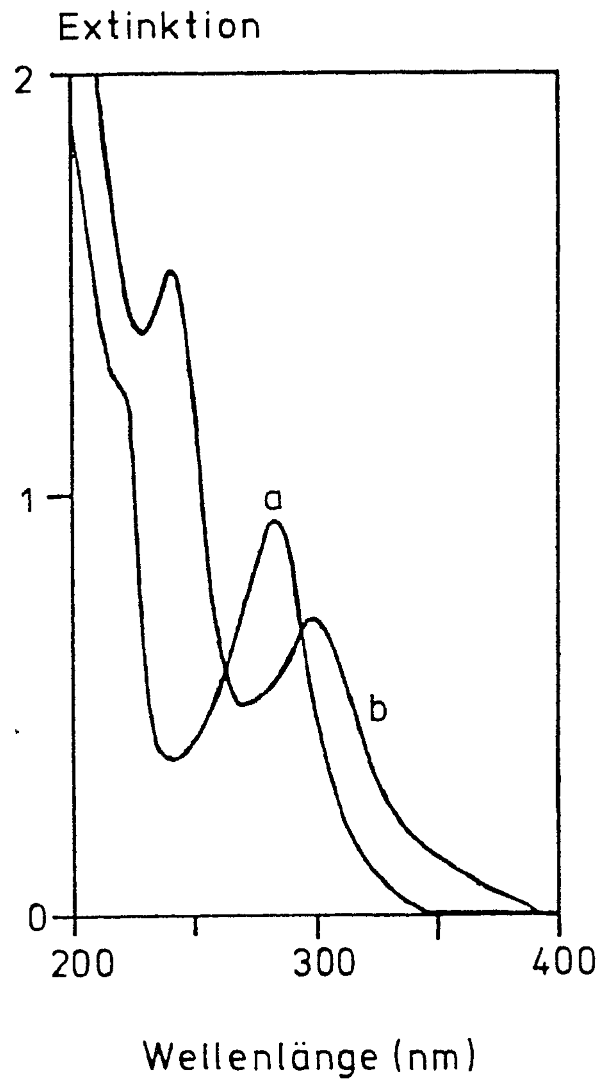
Extinktion
2
1
0
a
b
200
300
400
Wellenlänge (nm)

FIG. 5

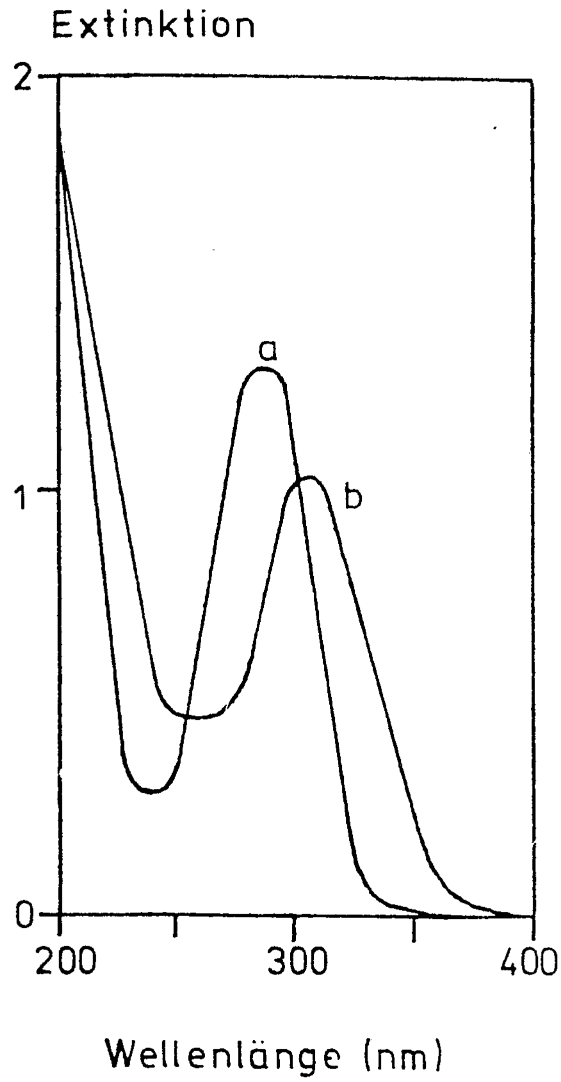
Extinktion
2
1
0
a
b
200
300
400
Wellenlänge (nm)

FIG. 6

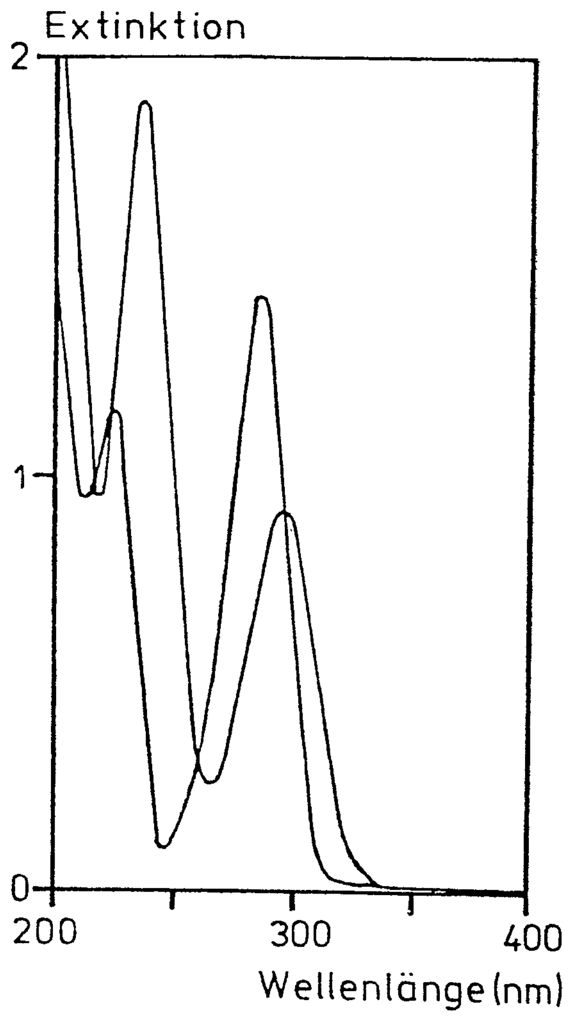
Extinktion
2
1
0
200
300
400
Wellenlänge (nm)

FIG.7

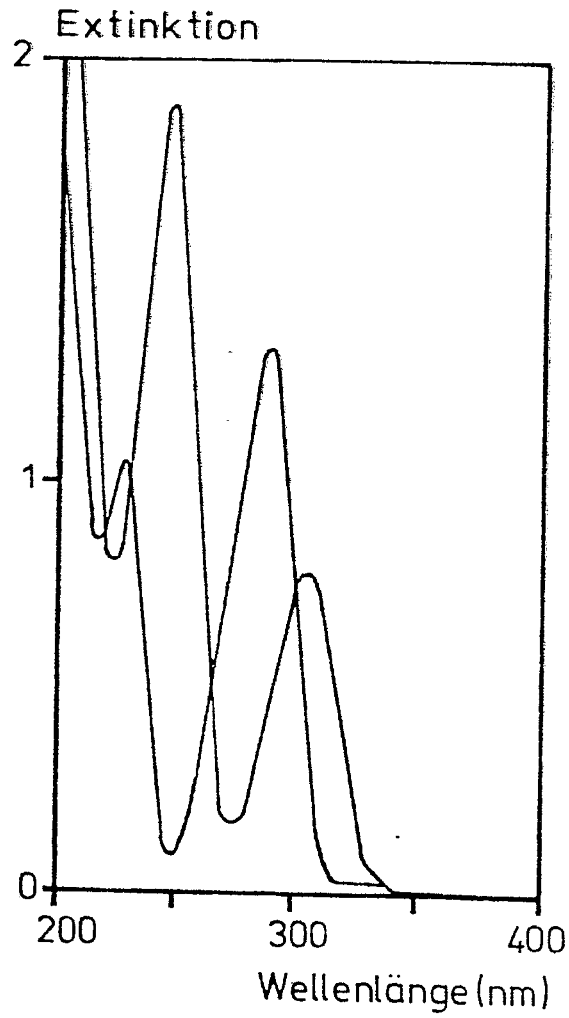
Extinktion
2
1
0
200
300
400
Wellenlänge (nm)

FIG.8

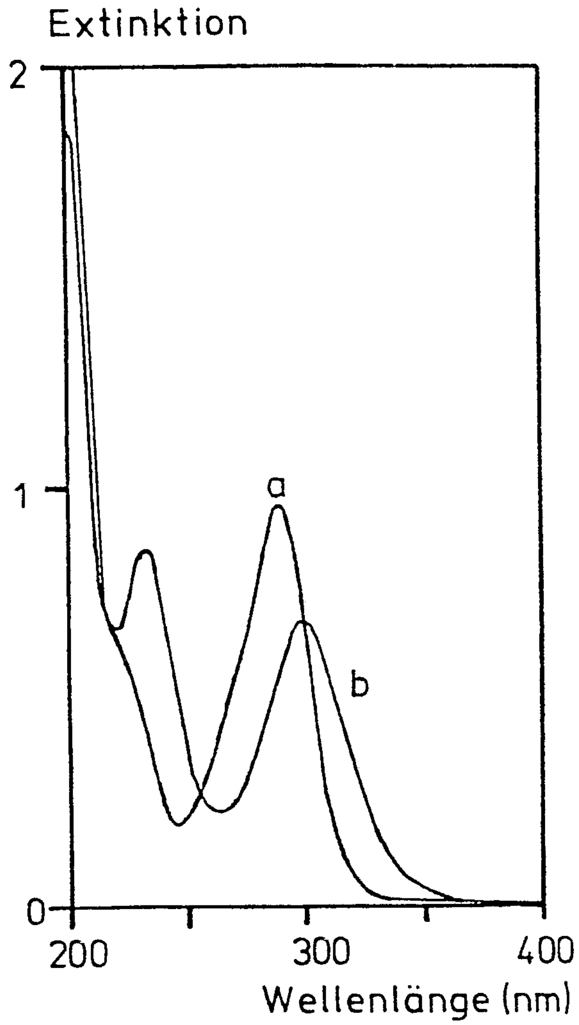
Extinktion
2
1
0
a
b
200
300
400
Wellenlänge (nm)

FIG. 9

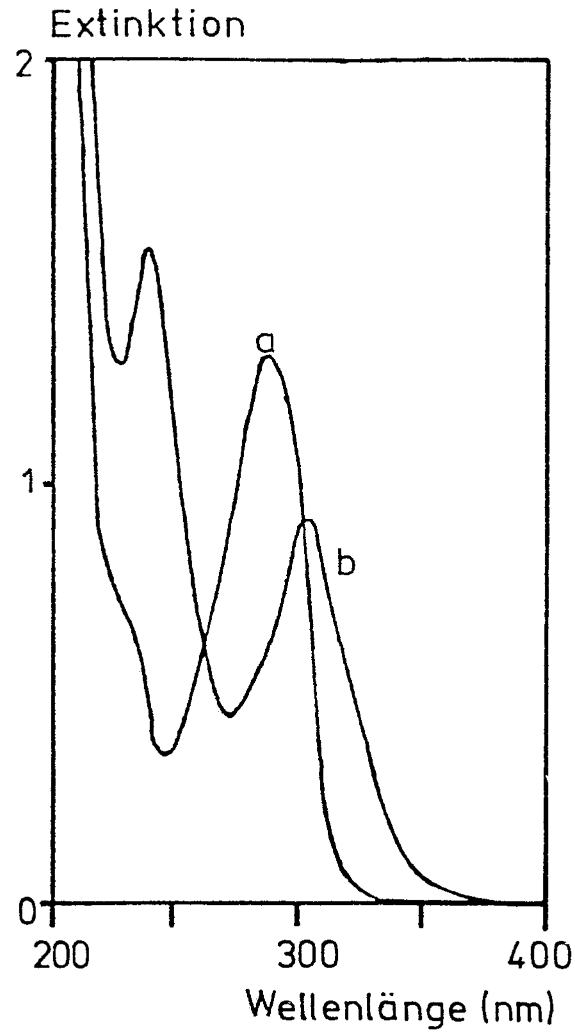
Extinktion
2
1
0
a
b
200
300
400
Wellenlänge (nm)

FIG. 10

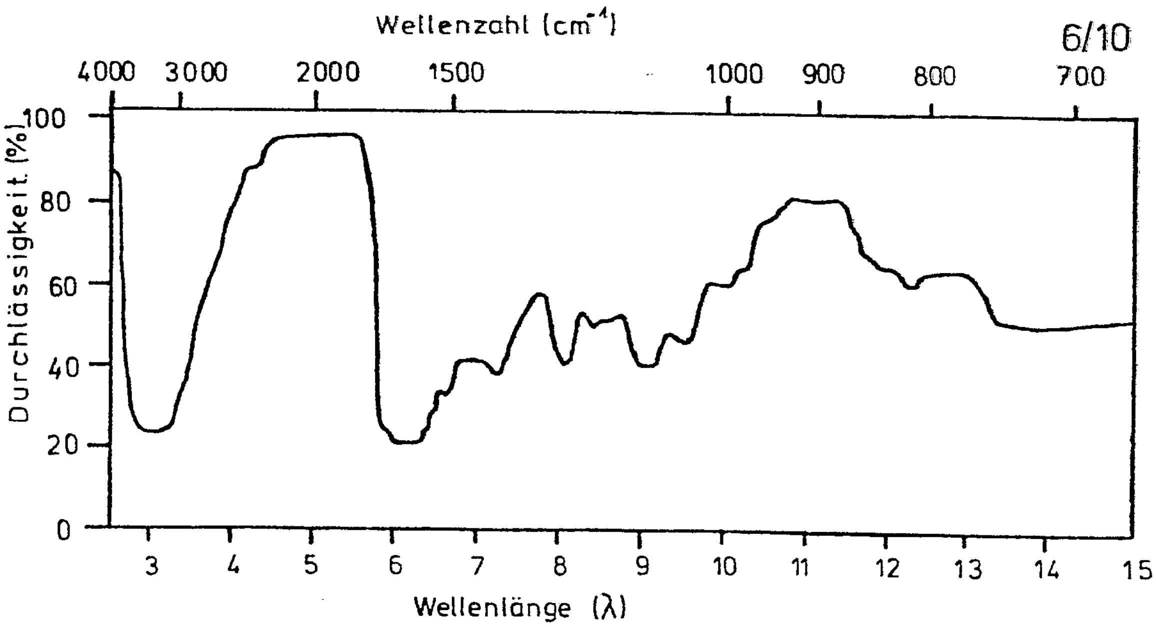
Wellenzahl (cm⁻¹)
4000 3000 2000 1500 1000 900 800 700
Durchlässigkeit (%)
100 80 60 40 20 0
3 4 5 6 7 8 9 10 11 12 13 14 15
Wellenlänge (λ)

FIG. 11

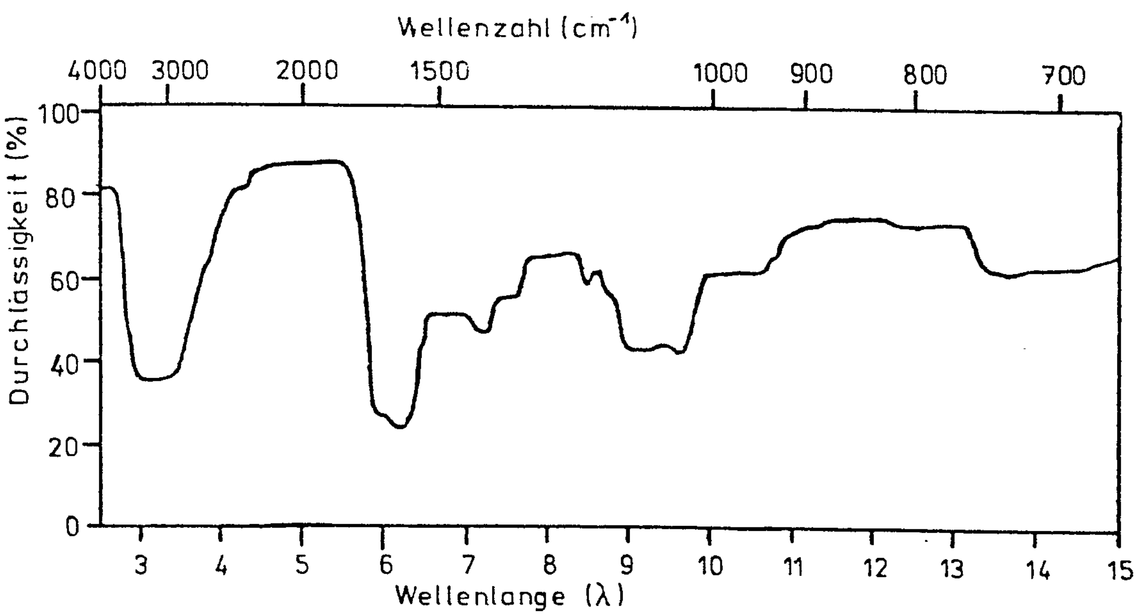
Wellenzahl (cm⁻¹)
4000 3000 2000 1500 1000 900 800 700
Durchlässigkeit (%)
100 80 60 40 20 0
3 4 5 6 7 8 9 10 11 12 13 14 15
Wellenlange (λ)

FIG. 12

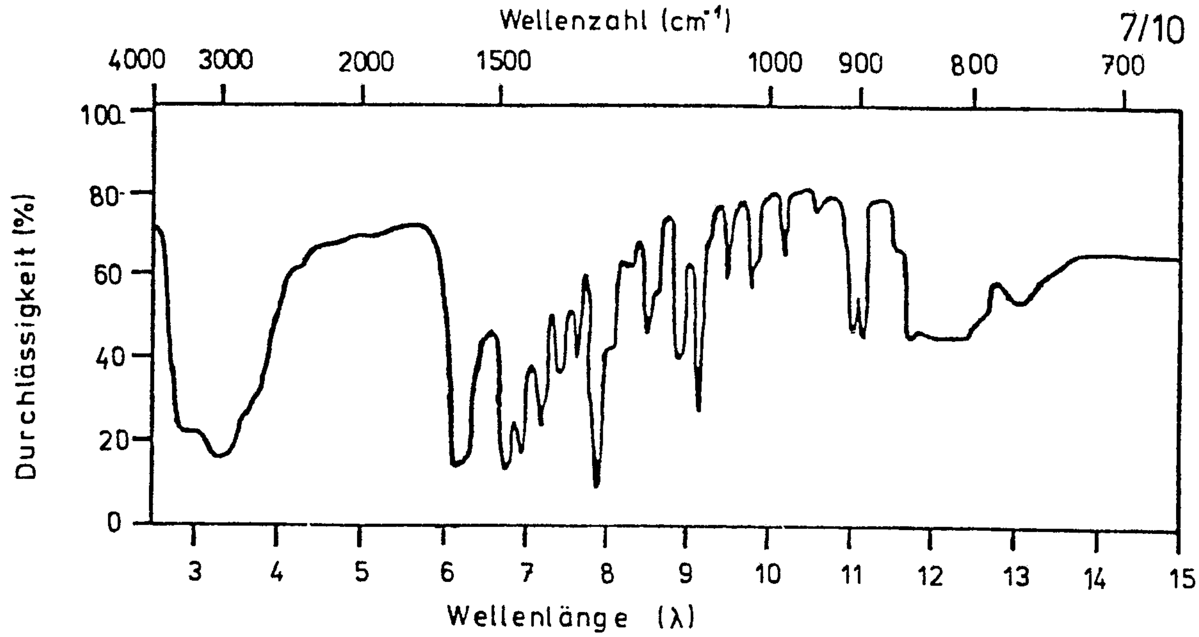
Wellenzahl (cm⁻¹)
4000
3000
2000
1500
1000
900
800
700
Durchlässigkeit (%)
100
80
60
40
20
0
3
4
5
6
7
8
9
10
11
12
13
14
15
Wellenlänge (λ)

FIG.13

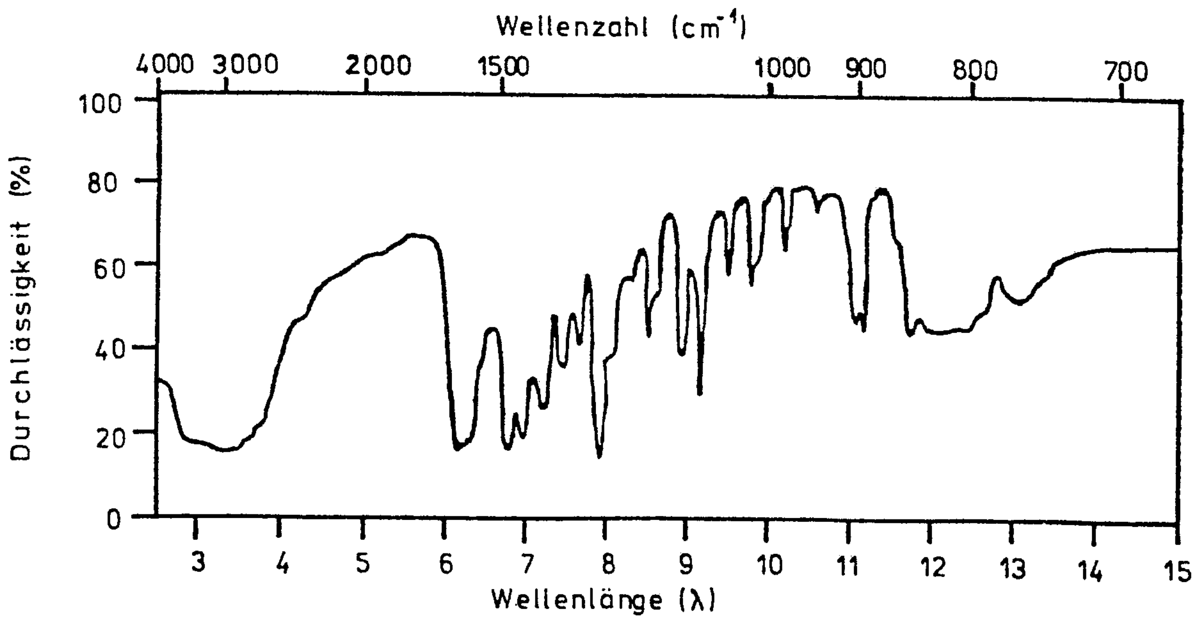
Wellenzahl (cm⁻¹)
4000
3000
2000
1500
1000
900
800
700
Durchlässigkeit (%)
100
80
60
40
20
0
3
4
5
6
7
8
9
10
11
12
13
14
15
Wellenlänge (λ)

FIG.14

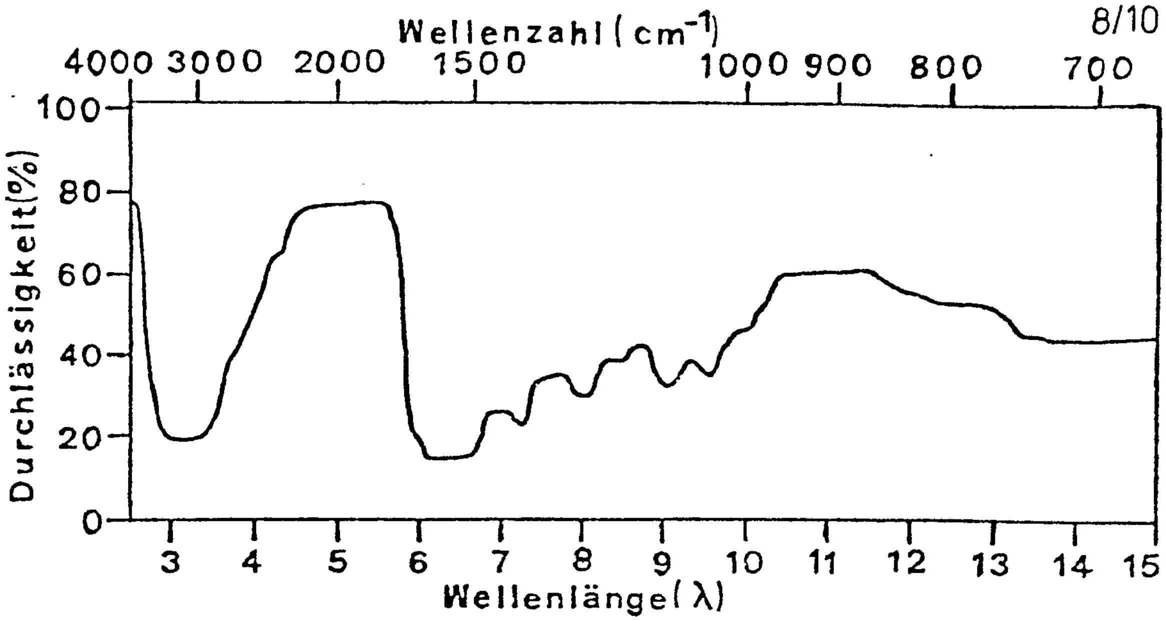
Wellenzahl (cm-1)
4000 3000 2000 1500 1000 900 800 700
100
80
60
40
20
0
Durchlässigkeit(%)
3 4 5 6 7 8 9 10 11 12 13 14 15
Wellenlänge(λ)

FIG. 15

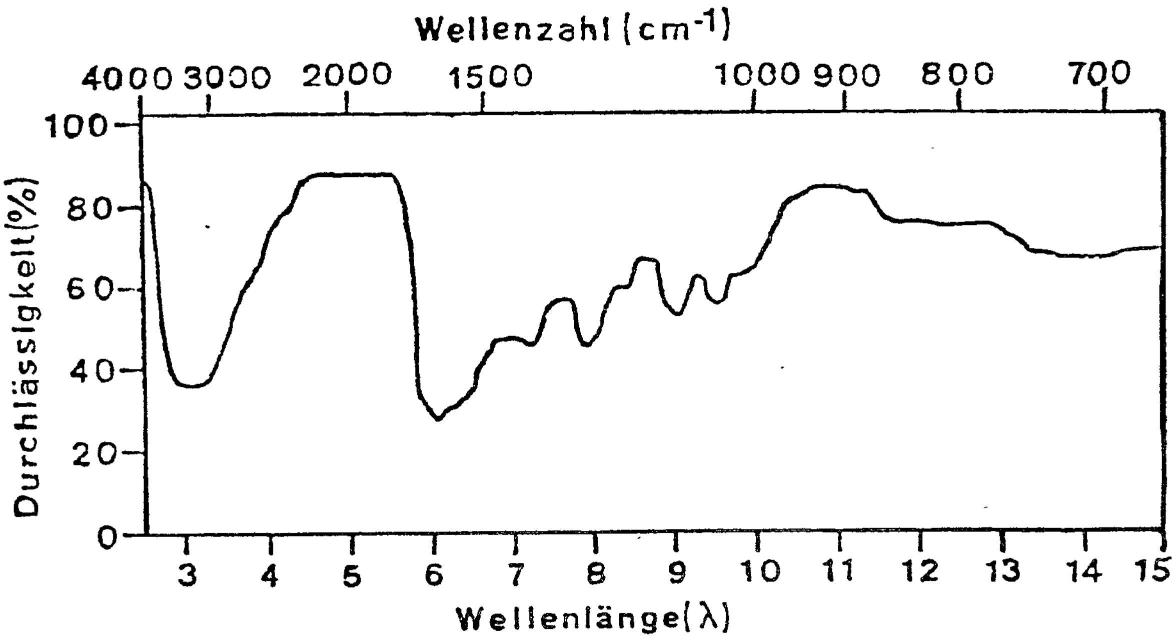
Wellenzahl (cm-1)
4000 3000 2000 1500 1000 900 800 700
100
80
60
40
20
0
Durchlässigkeit(%)
3 4 5 6 7 8 9 10 11 12 13 14 15
Wellenlänge(λ)

FIG.16

FIG. 17

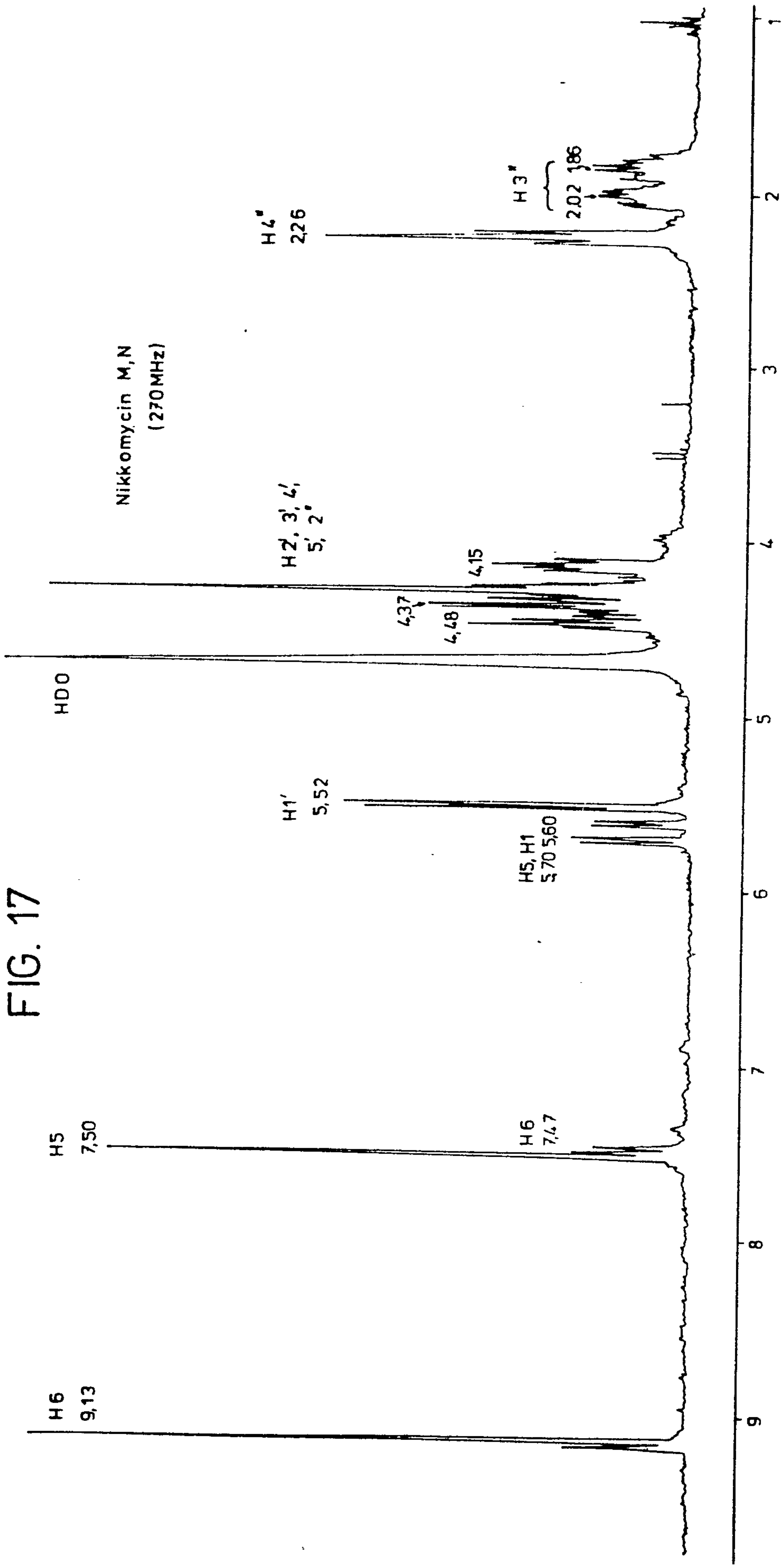
Nikkomycin M,N
(270MHz)
H6
9,13
H5
7,50
H6
7,47
H5, H1
5,70 5,60
H1'
5,52
HDO
H2', 3', 4',
5', 2''
4,37
4,48
4,15
H4''
2,26
H3''
2,02 1,86
1
2
3
4
5
6
7
8
9

FIG. 18

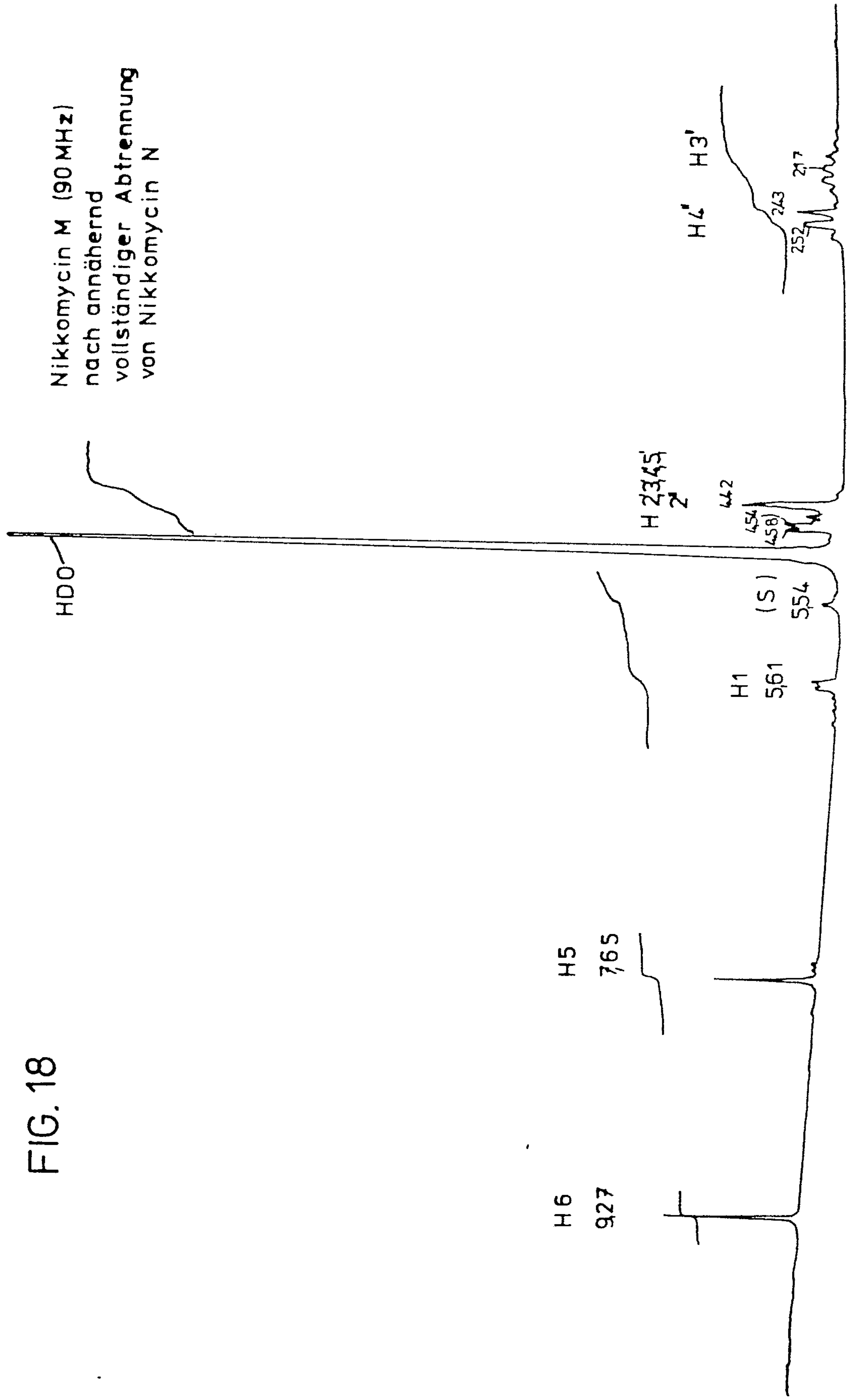
Nikkomycin M (90 MHz)
nach annähernd
vollständiger Abtrennung
von Nikkomycin N
HDO
H 2,3,4,5'
2''
4,42
4,54
4,58
H4'
H3'
2,52
2,43
2,17
(S)
5,54
H1
5,61
H5
7,65
H6
9,27